# FASCICULE DE BREVET EUROPEEN

(11) **EP 0 977 844 B1**
(45) Date de publication et mention de la délivrance du brevet: **28.11.2007**
(21) Numéro de dépôt: 98908176.5
(22) Date de dépôt: 11.02.1998
(51) Int. Cl.: C12N 15/12, C07K 14/705, C07K 16/28, A61K 38/17, G01N 33/68

(54) **CANAL CATIONIQUE NEURONAL DE MAMMIFERE SENSIBLE A L'ACIDITE, SON CLONAGE ET SES APPLICATIONS**
SÄUREEMPFINDLICHER NEURONALER SÄUGETIERKATIONENKANAL, DESSEN KLONIERUNG UND ANWENDUNG
MAMMAL NEURONAL ACID SENSING CATIONIC CHANNEL, CLONING AND APPLICATIONS THEREOF

(30) Priorité: 11.02.1997 FR 9701574; 28.07.1997 FR 9709587
(43) Date de publication de la demande: 09.02.2000
(73) Titulaire: CENTRE NATIONAL DE LA RECHERCHE SCIENTIFIQUE (CNRS), 75016 Paris (FR)
(72) Inventeur: WALDMANN, Rainer, F-83600 Les Adrets de l'Esterel (FR); BASSILANA, Frédéric, F-06000 Nice (FR); LAZDUNSKI, Michel, 06000 Nice (FR); CHAMPIGNY, Guy, décédé (FR); HEURTEAUX, Catherine, F-06600 Antibes (FR); LINGUEGLIA, Eric, F-06000 Nice (FR)
(74) Mandataire: Breese, Pierre
(86) Numéro de dépôt international: PCT/FR1998/000270
(87) Numéro de publication internationale: WO 1998/035034

(56) Documents cités:
- WALDMANN R ET AL: "THE MAMMALIAN DEGENERIN MDEG, AN AMILORIDE-SENSITIVE CATION CHANNEL ACTIVED BY MUTATIONS CAUSING NEURODEGENERATION IN CAENORHABDITIS ELEGANS" JOURNAL OF BIOLOGICAL CHEMISTRY, vol. 271, no. 18, 3 mai 1996, pages 10433-10436, XP002051361 cité dans la demande
- COREY D P ET AL: "MECHANOSENSATION AND THE DEG/ENAC ION CHANNELS" SCIENCE, vol. 273, no. 5273, 19 juillet 1996, page 323/324 XP002051360
- GILBERTSON, TIMOTHY A. ET AL: "Proton currents through amiloride-sensitive sodium channels in isolated hamster taste cells: Enhancement by vasopressin and cAMP" NEURON (1993), 10(5), 931-42 CODEN: NERNET;ISSN: 0896-6273, XP002068540
- PRICE MP ET AL: "Cloning and expression of a novel human brain Na+ channel." J BIOL CHEM, APR 5 1996, 271 (14) P7879-82, UNITED STATES, XP002068541
- EMBL databank Accession number w62694 09-JUN-1996 Marra M et al. XP002068546 cité dans la demande
- AKAIKE N ET AL: "Proton-induced current in neuronal cells." PROG NEUROBIOL, MAY 1994, 43 (1) P73-83, ENGLAND, XP002068567
- WALDMANN R ET AL: "A proton-gated cation channel involved in acid-sensing." NATURE, MAR 13 1997, 386 (6621) P173-7, ENGLAND, XP002068589
- BASSILANA F ET AL: "The acid-sensitive ionic channel subunit ASIC and the mammalian degenerin MDEG form a heteromultimeric H+-gated Na+ channel with novel properties." J BIOL CHEM, NOV 14 1997, 272 (46) P28819-22, UNITED STATES, XP002068543
- LINGUEGLIA E ET AL: "A modulatory subunit of acid sensing ion channels in brain and dorsal root ganglion cells." J BIOL CHEM, NOV 21 1997, 272 (47) P29778-83, XP002068544
- BARBRY P ET AL: "Molecular biology of Na+ absorption." AM J PHYSIOL, SEP 1997, 273 (3 PT 1) PG571-85, UNITED STATES, XP002068545
- GARCIA-ANOVEROS J ET AL: "BNAC1 AND BNAC2 CONSTITUTE A NEW FAMILY OF HUMAN NEURONAL SODIUM CHANNELS RELATED TO DEGENERINS AND EPITHELIAL SODIUM CHANNELS" PROCEEDINGS OF THE NATIONAL ACADEMY OF SCIENCES OF USA, vol. 94, no. 4, 18 février 1997, pages 1459-1464, XP002051359

## Description

La présente invention concerne une nouvelle famille de canaux ioniques de mammifère, notamment humain, sensible à l'acidité. Elle concerne plus particulièrement l'identification et la caractérisation moléculaire, chez l'homme et le rat, d'un nouveau canal cationique activé par les protons, dénommé ci-après "ASIC" pour désigner les termes anglais "Acid Sensing Ionic Channel". Le canal ASIC constitue le premier membre d'un groupe de canaux cationiques, appartenant à la famille des canaux sodium de dégénérine sensible à l'amiloride (6, 11-14), qui est activé transitoirement par une acidification extracellulaire.

La sensibilité à l'acide est associée à la fois à la nociception (1) et à la transduction du goût (2). La stimulation de neurones sensoriels par les acides revêt une grande importance, car l'acidité accompagne de nombreuses situations inflammatoires et ischémiques douloureuses. La douleur causée par les acides est interprétée comme étant médiée par des canaux cationiques présents au niveau des neurones sensoriels, et qui sont activés par les protons (3-5). Les propriétés biophysiques et pharmacologiques des canaux ASIC de l'invention sont proches de celles des canaux cationiques activés par les protons décrits dans les neurones sensoriels (3, 15, 16). Toutefois, comme cela apparaîtra dans la description ci-après, il n'a été à ce jour jamais décrit de canaux ioniques activés par un ligand plus simple que les canaux ASIC.

La présente invention a donc pour objet une protéine constituant un canal cationique neuronal sensible à l'amiloride et activé par les protons dont la séquence en acides aminés est représentée dans la liste de séquences en annexe sous le numéro SEQ ID No : 1 ou sous le numéro SEQ ID No : 2, et qui est sensiblement identique à celle du canal ASIC de rat, désigné ASIClA, représentée dans la liste de séquences en annexe sous le numéro SEQ ID No : 1.

Un autre exemple est la protéine constituant un canal cationique de dégénérine dénommé "MDEG" (14) ou "BNaCI" (20) ou encore désigné ci-après "ASIC2A" ou "MDEG1" dont la séquence en acides aminés est représentée dans la liste de séquences en annexe sous le numéro SEQ ID No : 3. MDEG a été décrit comme un canal cationique de mammifère sensible à l'amiloride qui est activé par des mutations responsables de neurodégénéréscence avec les dégénérines de *C. elegans.* Le canal MDEG est un parent structural du canal ASIC, dont la séquence en acides aminés présente environ 67% d'homologie avec la séquence du canal ionique MDEG. Toutefois, les propriétés électrophysiologiques de ces deux canaux sont différentes car ils ne sont pas activés par les mêmes changements de pH. Ainsi, la gamme de sensibilité de MDEG (EC₅₀ = 4,05) est différente de celle de ASIC (EC₅₀ = 6,2).

Il a été montré que le canal MDEG est activé par les mêmes mutations que celles causant une dégénérescence neuronale chez *C. elegans.* Ainsi, comme les mutants de dégénérine de *C. elegans* hyperactifs, les mutants actifs de MDEG sont responsables d'une mort cellulaire, indiquant que l'acquisition de fonction par ce canal ionique neuronal serait impliquée dans plusieurs formes de dégénérescence neuronale de mammifère et notamment humaine. Mais aucune fonction physiologique normale de MDEG n'était connue jusqu'à la mise en évidence de son activation par les protons conformément aux canaux cationiques de la présente invention.

D'autres exemples de protéines constituant un canal cationique neuronal sensible à l'amiloride et activé par les protons sont donnés ci-après :
- Un canal désigné ASIC1B dont la séquence de 559 acides aminés est représentée dans la liste de séquence en annexe sous le numéro SEQ ID NO : 4. ASIC1B est un variant dépissage du canal ASIC1A cloné à partir du cerveau de rat par PCR dégénérée. Les premiers 185 acides sont remplacés par une nouvelle séquence de 218 amino acides qui est soulignée dans la SEQ ID NO : 4.
- Un canal désigné DRASIC dont la séquence de 533 acides aminés est représentée dans la liste de séquence en annexe sous le numéro SEQ ID NO : 5. DRASIC a été clone à partir des neurones sensoriels de rat en utilisant une séquence partielle dans les banque de données ("Expressed Sequence Tag" avec le numéro d'accession W62694). Les propriétés de DRASIC sont les suivantes :
- Il est exprimé dans les neurones sensoriels mais pas dans le cerveau.
- Son expression dans les oocytes de Xénope ou dans des cellules de mammifère permet d'enregistrer un courant sodium activé par le proton qui présente deux composantes : une composante s'activant et s'inactivant rapidement et une composante s'activant plus lentement et ne s'inactivant pas. Les deux composantes sont sélectives pour le Na⁺. Un canal cationique activé par le proton et ne s'inactivant pas a été impliqué dans la sensation de douleur prolongée causée par une acidose.

L'invention concerne aussi un canal cationique hybride constitué de l'association d'une première protéine constituant un canal ionique activé ou non par les protons selon l'invention avec une seconde protéine constituant un canal ionique activé par les protons. Avantageusement, ladite seconde protéine est aussi une protéine constituant un canal ionique activé par les protons selon l'invention. A titre d'exemple d'une telle association, on peut citer l'association de la protéine du canal ASIC1A ou ASIC2A ou DRASIC avec la protéine du canal MDEG1, permettant de former un canal hybride présentant une troisième gamme de sensibilité au pH (avec ASIC : EC₅₀ - 4,8). Un autre exemple d'un tel canal hybride est l'association de la protéine des canaux ASIC1A, ASIC1B, MEDG1 ou DRASIC avec la protéine du canal MDEG2.

MDEG2 est un canal qui a été cloné à partir du cerveau de rat en utilisant une séquence partielle de souris accessible dans les banques de données ("Expressed Sequence Tag avec le numéro d'accession W50528") et dont la séquence de 563 acides aminés est représentée dans la liste de séquence en annexe sous le numéro SEQ ID NO : 6.

MDEG2 est un variant d'épissage de MDEG1. Les premier 185 amino acides sont remplacés par une nouvelle séquence de 236 amino acides qui est soulignée dans la SEQ ID NO : 6. MDEG2 est exprimé dans le cerveau et dans les neurones sensoriels des ganglions de la racine dorsale.

MDEG2 exprimé seul dans les oocytes de Xénope ou dans des cellules de mammifères ne forme pas un canal cationique activé par le proton. Néanmoins, il peut s'associer avec MDEG1 ou DRASIC pour former des canaux hétéromultimériques activés par le proton avec des propriétés modifiées :
- Le pH d'activation du canal formé après la co-expression de MDEG1 et MDEG2 est diffèrent. Après expression dans les cellules COS, le courant n'a pas atteint sa valeur maximale à pH 3 alors que le courant induit par MDEG1 seul sature à un pH compris entre 4,5 et 4,0.
- Les cinétiques d'inactivation et la sélectivité ionique du canal formé après la co-expression de MDEG1 et MDEG2 sont clairement différentes de celles de MDEG1 seul. Un courant s'inactivant lentement et peu sélectif pour le Na+ et le K+ apparaît.
- Le courant sodique soutenu obtenu après expression de DRASIC devient non sélectif (il ne différencie plus le sodium et le potassium) quand MDEG2 est co-exprimé avec DRASIC. Cette nouvelle propriété est similaire à celle du canal cationique activé par le proton qui a été impliqué dans la sensation de douleur prolongée causée par une acidose. Il est très probable que DRASIC et MDEG2 fassent partie de ce canal.

Les homologies de séquences en acides aminés des protéines constituant les canaux ASIC1A, ASIC1B, cités selon l'invention sont données dans la tableau 1 ci-dessous.

**Tableau 1**

| Canaux | ASIC 1B | ASIC lA | MEDG2 | MDEG1 | DRASIC |
|---|---|---|---|---|---|
| ASIC1B | 100 | 80 | 56 | 61 | 52 |
| ASIC1A | | 100 | 59 | 68 | 53 |
| MDEG2 | | | 100 | 78 | 48 |
| MDEG1 | | | | 100 | 51 |
| DRASIC | | | | | 100 |

Des anticorps poly ou monoclonaux dirigés contre au moins une protéine constituant un canal ionique de l'invention et/ou contre un canal hybride ci-dessus, peuvent être préparés par les méthodes classiques décrites dans la littérature. Ces anticorps sont utiles pour rechercher la présence des canaux ioniques de l'invention dans différents tissus humains ou animaux, mais ils peuvent aussi trouver des applications dans le domaine thérapeutique pour inhiber ou activer *in vivo,* grâce à leur spécificité, un canal ASIC et/ou ses dérivés.

La présente invention a aussi pour objet une molécule d'acide nucléique comprenant ou constituée par une séquence nucléique codant pour une protéine constituant un canal cationique neuronal sensible à l'amiloride et activé par les protons. Plus particulièrement l'invention concerne une molécule d'acide nucléique comprenant au moins une séquence codant pour la protéine constituant le canal ASIC dont la séquence en acides aminés est représentée dans la liste de séquences en annexe sous le numéro SEQ ID No : 1 ou pour un dérivé fonctionnellement équivalent de cette protéine. Une molécule d'ADN comprenant la séquence codant pour la protéine ASIC est celle représentée dans la liste de séquences en annexe sous le numéro SEQ ID NO:1 ou sa séquence complémentaire. Une autre molécule d'ADN selon l'invention est celle représentée dans la liste de séquence en annexe sous le numéro SEQ ID NO : 2 ou sous le numéro SEQ ID NO : 3, ou leur séquence complémentaire.

Une molécule d'ADN comprenant la séquence codant pour la protéine ASIC1B est celle de 3647 pb représentée dans la liste de séquence en annexe sous le numéro SEQ ID NO : 4 ou sa séquence complémentaire. Plus particulièrement l'invention concerne la séquence nucléique comprise entre les nucléotides 109 et 1785 de la séquence représentée dans la liste de séquence en annexe sous le numéro SEQ ID NO : 4 ou sa séquence complémentaire.

Une molécule d'ADN codant pour la protéine DRASIC est celle de 1602 pb représentée dans la liste de séquence en annexe sous le numéro SEQ ID NO : 5 ou sa séquence complémentaire.

Une molécule d' ADN comprenant la séquence codant pour la protéine MDEG2 est celle de 1602 pb représentée dans la liste de séquence en annexe sous le numéro SEQ ID NO : 6 ou sa séquence complémentaire.

L'invention concerne également un vecteur comprenant au moins une molécule d'acide nucléique précédente, avantageusement associée à des séquences de contrôle adaptés, ainsi qu'un procédé de production ou d'expression dans un hôte cellulaire d'une protéine constituant un canal ionique selon l'invention. La préparation de ces vecteurs ainsi que la production ou l'expression dans un hôte des canaux de l'invention peuvent être réalisées par les techniques de biologie moléculaire et de génie génétique bien connues de l'homme du métier.

A titre d'exemple, un procédé de production d'une protéine constituant un canal cationique selon l'invention consiste :
- à transférer une molécule d'acide nucléique de l'invention ou un vecteur contenant ladite molécule dans un hôte cellulaire,
- à cultiver ledit hôte cellulaire dans des conditions permettant la production de la protéine constituant le canal cationique,
- à isoler, par tous moyens appropriés les protéines constituant les canaux ioniques de l'invention.

A titre d'exemple, un procédé d'expression d'un canal ionique selon l'invention consiste :
- à transférer une molécule d'acide nucléique de l'invention ou un vecteur contenant ladite molécule dans une cellule,
- à cultiver ledit hôte cellulaire dans des conditions permettant l'expression de canaux ioniques de l'invention.

L'hôte cellulaire mis en oeuvre dans les procédés précédents peut être choisi parmi les procaryotes ou les eucaryotes et notamment parmi les bactéries, les levures, les cellules de mammifères, de plantes ou d'insectes.

Le vecteur utilisé est choisi en fonction de l'hôte dans lequel il sera transféré; il peut s'agit de tout vecteur comme un plasmide.

L'invention concerne donc aussi les cellules transformées exprimant les canaux ASIC et/ou ses dérivés comme MDEG obtenues conformément aux procédés précédents. Ces cellules sont utiles pour le criblage de substances capables de moduler la perception de l'acidité, tant en ce qui concerne la nociception que la transduction du goût. Ce criblage est effectué en mettant en contact des quantités variables d'une substance à tester avec des cellules exprimant les canaux ASIC, puis en mesurant, par tous moyens appropriés, les effets éventuels de ladite substance sur les courants desdits canaux. Des techniques électrophysiologiques permettent également ces études et font aussi l'objet de la présente invention dès lors qu'elles mettent en oeuvre les canaux ASIC ou leurs dérivés. Ces criblages permettent d'identifier de nouveaux médicaments utiles dans le traitement ou la prévention de la douleur. Ils permettent également de rechercher des agents modulateurs du goût acide. En outre, ils permettent de rechercher des bloqueurs qui sont susceptibles d'inhiber des neurodégénéresences provoquées par hyperexpression de ces canaux. Ces médicaments, isolés et détectés grâce aux procédés ci-dessus, font également partie de l'invention. Les canaux ASIC ont en effet des propriétés de sélectivité ionique, notamment en ce qui concerne leur perméabilité sélective au sodium, potassium et calcium, qui les destinent à avoir des propriétés excitotoxiques lorsqu'ils sont hyperstimulés.

Une protéine constituant un canal ionique neuronal ASIC peut être aussi utile pour la fabrication de médicaments destinés à traiter ou prévenir des pathologies impliquant la perception douloureuse de l'acidité qui intervient dans les maladies inflammatoires, les ischémies et dans un certain nombre de tumeurs. L'invention concerne donc aussi les composition pharmaceutiques comprenant comme principe actif au moins une protéine constituant un canal ionique selon l'invention.

Une molécule d'acide nucléique codant pour une protéine constituant un canal ASIC ou un dérivé de celui-ci, ou un vecteur comprenant cette molécule d'acide nucléique ou encore une cellule exprimant des canaux ASIC, sont aussi utiles pour la préparation d'animaux transgéniques. I1 peut s'agir d'animaux sur-exprimant lesdits canaux, mais surtout d'animaux dit "knock out", c'est à dire présentant une déficience en ces canaux; ces animaux transgéniques sont préparés par des méthodes connues de l'homme du métier, et permettent de disposer de modèles vivants pour l'étude de pathologies animales associées aux canaux ASIC.

Les molécules d'acide nucléique de l'invention ou les cellules transformées par ladite molécule sont donc susceptibles d'être utilisées dans des stratégies de thérapie génique afin de compenser une déficience des canaux ASIC au niveau de un ou plusieurs tissus d'un patient. L'invention concerne donc aussi un médicament comprenant des molécules d'acide nucléique de l'invention ou de cellules transformées par lesdites molécules pour le traitement de pathologie impliquant les canaux ASIC et leurs dérivés.

Outre la propriété d'être activé par les protons et les applications décrites ci-dessus qui en résultent dans le domaine de la perception de l'acidité, le canal ASIC, du fait de sa parenté structurale avec le canal MDEG, est susceptible de se comporter comme une dégénérine neuronale à la suite de mutation.

La mort de certains neurones est caractéristique de plusieurs types de dégénérescences neuronales telles que les maladies d'Alzheimer, d'Huntington, de Parkinson, la sclérose latérale amyotrophique, l'ataxie cérébelleuse. Seuls quelques gènes déficients sont connus et plusieurs restent encore à identifier. Le réseau neuronal primitif du nématode *C. elegans* constitue un bon modèle du développement et de la mort neuronal. La dégénérescence héréditaire chez *C. elegans* peut être due à des mutations des dégénérines deg-1, mec-4 et mec 10. Les homologies avec les sous-unités du canal sodium sensible à l'amiloride, le produit d'expression fonctionnel des chimères mec-4 du canal sodium épithélial, suggèrent que les dégénérines sont des canaux ioniques dont l'acquisition de fonction est la cause de dégénérescence neuronale.

La présente invention concerne donc aussi les applications du canal ASIC pour l'étude de ces modifications pathologiques susceptibles de conduire à des dégénérescences. Les techniques mises en oeuvre pour ces applications, par exemple pour le criblage de drogues, sont similaires à celles décrites précédemment pour la recherche d'agents modulateurs du goût ou d'analgésiques.

En outre, une protéine constituant un canal ionique neuronal ASIC, un agoniste ou un antagonsite de celle-ci, peuvent être aussi utiles pour la fabrication de médicaments destinés à traiter ou prévenir des pathologies impliquant une dégénéréscence neuronale cérébrale. L'invention concerne donc aussi les compositions pharmaceutiques comprenant comme principe actif au moins une de ces protéines éventuellement associée à un véhicule physiologiquement acceptable.

Plus particulièrement, l'invention concerne une substance chimique ou biologique capable de modifier les courants d'un canal ionique et/ou un canal hybride selon l'invention pour la préparation d'un médicament capble de moduler la perception de l'acidité, tant en ce qui concerne la nociception que la transduction du goût, chez un sujet humain ou animal.

D'autres caractéristiques et avantages de l'invention apparaîtront dans la description qui suit se rapportant aux travaux de recherche ayant mené à la mise en évidence et à la caractérisation du canal ASIC, et dans laquelle il sera fait référence aux séquences et dessins en annexe dans lesquels :
- SEQ ID NO : 1 représente la séquence de 526 acides aminés de la protéine du canal ASIC déduite de la séquence de l'ADNc de rat.
- SEQ ID NO : 2 représente la séquence partielle de 514 acides aminés de la protéine du canal ASIC déduite de la séquence partielle de l'ADNc humain.
- SEQ ID NO : 3 représente la séquence de 512 acides aminés de la protéine du canal MDEG déduite de la séquence de l'ADNc humain.
- SEQ ID NO : 4 représente la séquence de 559 acides aminés de la protéine du canal ASIC1B ainsi que la séquence d'une molécule d'ADN comprenant la séquence codant pour cette protéine.
- SEQ ID NO : 5 représente la séquence de 533 acides aminés de la protéine du canal DRASIC et la séquence d'ADN codant pour cette protéine.
- SEQ ID NO : 6 représente la séquence de 563 acides aminés de la protéine du canal MDEG2 ainsi que la séquence d'une molécule d'ADN comprenant la séquence codant pour cette protéine.

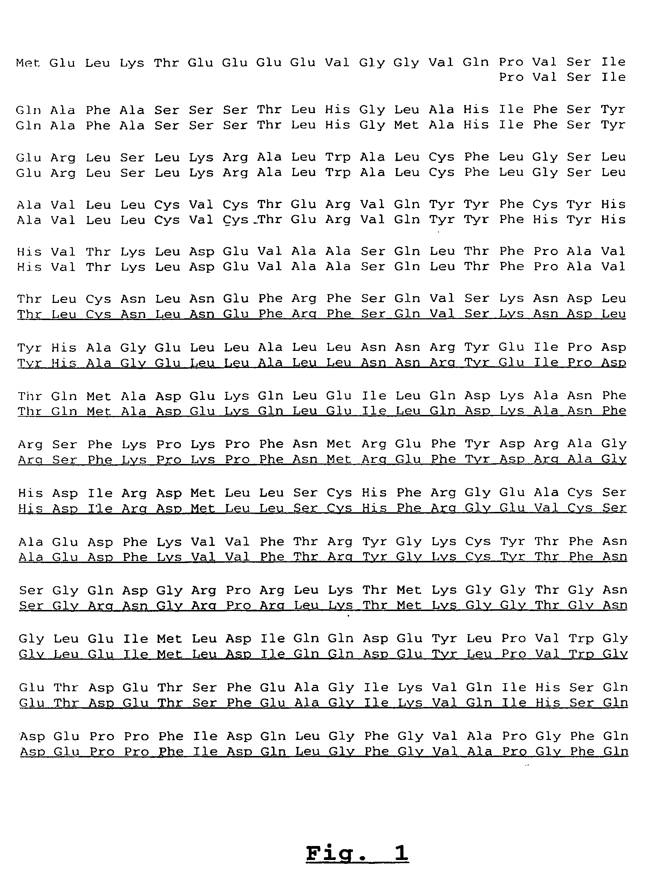

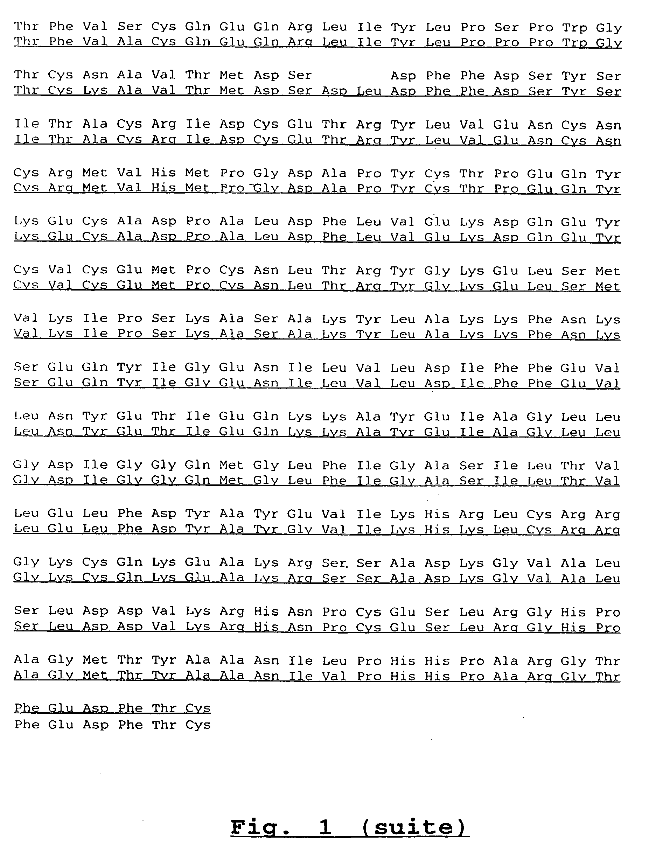

- La figure 1 représente l'alignement des séquences des protéines ASIC de rat (en haut) et humain (en bas) des séquences SEQ ID NO : 1 et SEQ ID NO : 2. La comparaison de ces séquences fait apparaître l'absence de 14 acides aminés au début de la phase codante humains par rapport à celle du rat.

La figure 2 représente la comparaison de la séquence de la protéine du canal ASIC avec la séquence d'autres canaux ioniques :
- MDEG (14), un canal cationique de mammifère qui est activé par des mutations responsables de neurodégérescences avec les dégénérines de *C. elegans.*
- FaNaCh (10), un peptide d'un canal sodium de *Helix aspersa* qui est activé par la FMRFamide.
- La dégénérine MEC-4 (12) de *C. elegans.*

Dans cette figure, les résidus identiques ou similaires à ceux de ASIC sont imprimés respectivement en blanc sur fond noir et en noir sur fond gris. Les régions supposées transmembranaires (MI, MII) d'ASIC sont marquées par des barres noires.

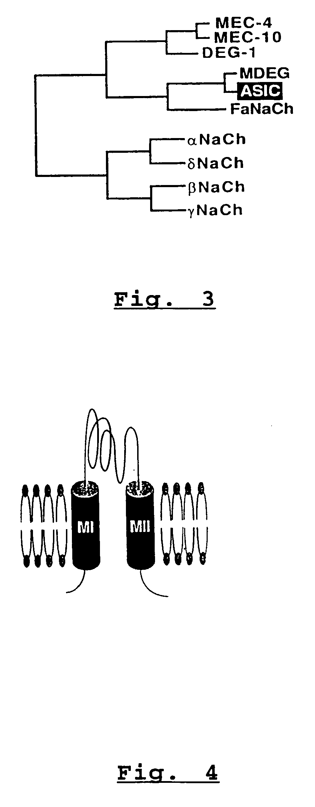

La figure 3 représente l'arbre phylogénétique des protéines des sous unités αNaCh, βNaCh, γNaCh, δNaCh du canal sodium sensible à l'amiloride et des dégénérines MEC-4, MEC-10 et DEG-1 de *C. elegans.*

La figure 4 représente la topologie qui est proposée pour cette dernière famille de canaux ioniques (30).

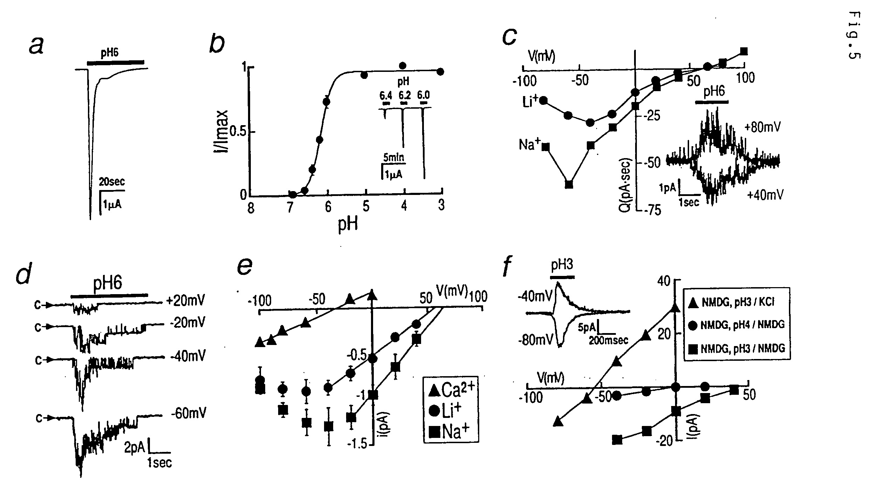

La figure 5 montre les propriétés biophysiques du canal ASIC1 activé par les protons.
- En a : les courants macroscopiques entrant enregistrés à -70 mV après des rapides changements du pH de pH 7,4 à pH 6.
- En b : La courbe dose réponse du pH extracellulaire. Le pH initial était de 7,4 et les points représentent les valeurs moyennes de 6 expériences. L'encart dans cette figure montre les réponses typiques à -70 mV.
- En c : les relations Q-V des patch "outside-out" avec 140 mM de Na⁺(■) ou de Li⁺(•) dans la solution du bain. Q est la charge transportée durant la transition de pH acide. L'encart dans cette figure montre les réponses typiques dans un milieu contenant du Na⁺.
- En d : les courants activés par les protons H⁺ enregistrés à différents potentiels dans un patch "outside-out" dans un milieu contenant du Na⁺.
- En e : les relations moyennes i-V mesurées à partir de patch "outside-out" avec 140 mM de Na⁺(■), 140 mM de Li⁺(•) ou 1,8 mM de Ca²⁺ ( ), en tant qu'ions perméables majoritaires dans les solutions externes; les potentiels d'inversion étaient respectivement de 65 mV, 58 mV et -34 mV.
- En f : le courant de protons à travers le canal ASIC1. Les relations entre le pic de courant et le voltage ont été mesurées à partir de patch "outside-out" dans une solution de Na⁺ libre, Ca²⁺ libre avec des pipettes contenant une solution de K⁺ libre, à pH 4 (•) et à pH 3 (■).( ) représentent les résultats obtenus dans les mêmes conditions que (■) mais avec du KCl dans la pipette. L'encart dans cette figure montre les réponses typiques enregistrées dans les conditions ( ).

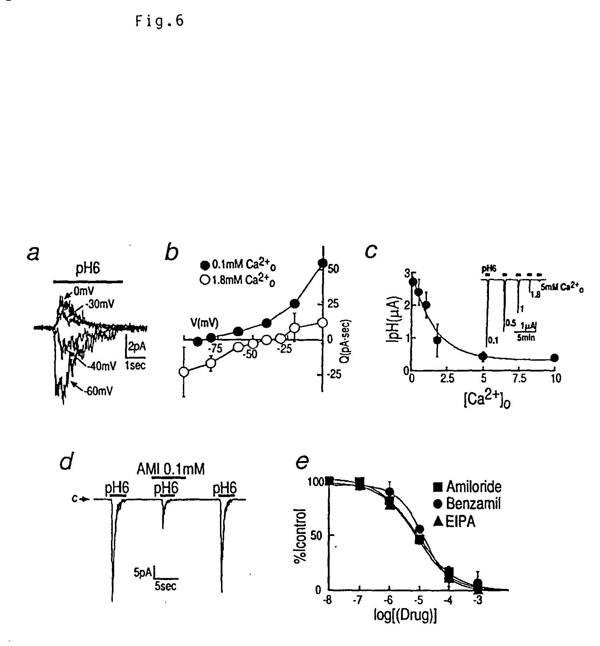

La figure 6 montre l'effet du Ca²⁺ et de l'amiloride sur le courant ASIC.
- En a : les courants activés par les protons H⁺ enregistrés à différents potentiels membranaires à partir d'un patch outside-out avec 1,8 mM de Ca²⁺ dans une solution de Na⁺ libre ; les courants se sont inversés à -35 mV.
- En b : Les relations Q-V moyennes à partir d'un patch outside-out enregistrées dans des solutions de Na⁺ libre contenant 1,8 mM de Ca²⁺ (o, potentiel d'inversion -34 mV) ou 0,1 mM de Ca²⁺ (•, potentiel d'inversion -80 mV).
- En c : L'effet du Ca²⁺ externe sur le pic macroscopique de courant entrant enregistré à -70 mV et activé par un changement rapide de pH de pH 7,4 à pH 6. L'encart dans cette figure montre les réponses typiques. Les points représentent les valeurs moyennes ± se de 5 oocytes.
- En d : L'effet de l'amiloride sur les courants activés par les protons H⁺ enregistré à 0 mV à partir d'un patch outside-out.
- En e : L'inhibition du courant macroscopique (induit par un changement de pH de pH 7,4 à pH 6) à -70 mV par l'amiloride et dérivés. Les points représentent les valeurs moyennes ± se de 5 oocytes.

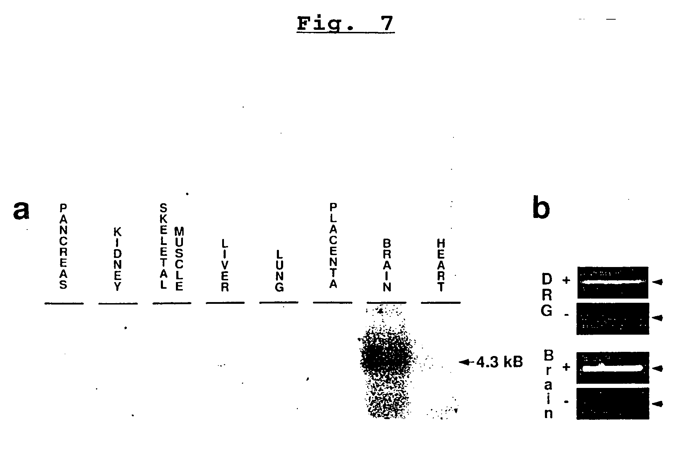

La figure 7 montre la distribution tissulaire de l'ARNm du canal ASIC.
- En a : L'analyse en Northern blot de l'expression ARNm du canal ASIC dans des tissus humains.
- En b : L'analyse en RT-PCR de l'expression de l'ARNm du canal ASIC dans le cerveau de rat et dans le ganglion de la racine dorsale (DRG). (+),(-) représentent respectivement les échantillons avec ou sans reverse transriptase. Des sections d'un gel d'agarose révélé au bromure d'éthidium 1%. Les flèches indiquent la taille escomptée (657 pb) du produit de PCR.

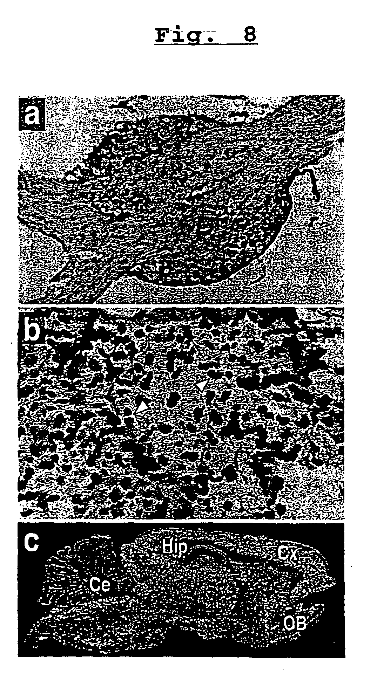

La figure 8 représente l'hybridation *in situ*.
- En a et b : L'hybridation de sections de 6 µm d'un ganglion de la racine dorsale d'un rat agé de 3 mois avec la sonde E marquée à la digoxigénine. En a : Une microphotographie à faible pouvoir éclairant (grossissement de 30 fois). En b : Une image à haute résolution (grossissement de 80 fois) de a. On note le marquage intense des neurones de petit diamètre (flèches). Des résultats similaires ont aussi été obtenus avec les sondes A, C et D.
- En c : La distribution de l'ARNm du canal ASIC dans le cerveau d'un rat adulte analysée par hybridation in situ avec l'oligonucléotide antisens C. Des résultats identiques ont été obtenus avec l'oligonucléotide B. Les couleurs indiquent l'abondance (rouge : haute expression ; bleu : non détectable). Les abréviations utilisées dans la figure sont les suivantes : Cer = Cerebellum ; Hip = Hyppocampe ; OB = Bulbe olfactif ; Cx = Cortex.

### I - Matériels et Méthodes.

### 1) Clonage du canal ASIC.

Les séquences conservées de la famille de canaux ioniques ASIC ont été utilisées pour préparer les amorces PCR de séquences suivantes :
TTYCCIGCIRTIACIITNTGYAAY, et
CAIARICCIAIITGNCCNCCDAWRTC.

Une banque d'ADNc de cerveau de rat (Stratagène #936515) a été hybridée avec le produit de PCR de 1 kB de cerveau de rat et des clones partiels ont été isolés. L'extrémité 5é de l'ADNc (202 bp) a été isolée par PCR après une ligation adaptée à l'ADNc double brin.

### 2) Electrophysiologie.

0,25 ng d'ARNc a été injecté dans des oocytes de *Xenopus laevis* et les microélectrodes d'enregistrement pour le voltage imposé et pour le patch-clamp ont été mises en place deux jours après l'injection. Les solutions de bains pour les enregistrements de patch outside-out et les pipettes pour les enregistrements de patch outside-out et de cellules totales, contenaient : 140 mM KCl (ou NMDG), 2 mM MgCl₂, 5 mM EGTA, 10 mM Hepes, pH 7,4 (avec KOH). Les pipettes pour les enregistrements de patch outside-out et les solutions de bains pour les enregistrements de patch outside-out et de cellules totales, contenaient: 140 mM NaCl (ou LiCl ou NMDGCl), 2 mM MgCl₂, 1,8 mM CaCl₂, 10 mM Hepes, pH 7,4 (ajusté avec HCl, NaOH, LiOH ou TMAOH). Les changements rapides de pH depuis le pH initial ont été obtenus par perfusion avec une solution de bain ajustée aux pH indiqués dans les figures. L'acidification intracellulaire des oocytes a été réalisée en injectant 50 nl de la solution interne à pH2 ou par perfusion et retrait d'un milieu de bain contenant 20 mM NH₄Cl. Aucun des courants enregistrés n'était contaminé par le courant Ca²⁺ sensible au Cl⁻ de l'oocyte de Xenopus. Les données ont été échantillonnées à 2 kHz et filtrées à 500 Hz pour l'nalyse (Logiciel Biopatch).

### 3) Analyse Nortern Blot, RT-PCR et hybridation in situ.

Le Northern blot a été obtenu auprès de la Société Clontech (Palo Alto, Ca) et contenait environ 2 µg d'ARN poly(A+) par ligne. Le blot a été hybridé avec un fragment du clone partiel humain (correspondant aux bases 270 à 764 du clone de rat) marqué au ³²P, à 65°C dans 6xSSC. Pour l'analyse RT-PCR, 5 µg de l'ARN total de cerveau de rat et 3 µg de ganglion de la racine dorsale ont été reverse transcrits et 1/30 de l'échantillon a été amplifié par 30 cycles de PCR avec les amorces de séquences ci-dessous :
ATTGCTCTTCCCATCTCTAT, et
TTCAAGGCCCATACCTAAGT.

Les contrôles négatifs ont été traités de façon identique, à l'exception de la reverse transcriptase qui n'a pas été ajoutée. Les oligonucléotides antisens correspondant aux base 70 à 114 (A), 215 à 248 (B), 1821 à 1859 (C), 1896 à 1940 (D) et l'ADN double brin correspondant aux base 1685 à 2672 ont été utilisés pour les hybridations in situ. Les sections de cerveau de rat adulte ont été hybridées avec les oligonucléotides B ou C dont l'extrémité était marquée au ³²P, pour une nuit à 37°C dans 50% formamide, 2xSSC, puis lavées à température ambiante dans 1xSSC. Le signal a été aboli par un excès 500 fois d'oligonucléotides non marqués. Les sections de ganglion de la racine dorsale ont été hybridées avec les oligonucléotides A, C ou D marqués par la digoxigénine(DIG)-dUTP et avec la sonde E marquée par DIG-d-UTP par PCR. Le marquage des sondes, la préparation des échantillons, l'hybridation et la visualisation des acides nucléiques DIG avec la phosphatase alcaline conjuguée à des anticorps anti-DIG ont été réalisés conformément aux protocoles du fournisseur (Boehringer Mannheim).

### 4) Analyse informatique.

L'alignement de séquences et l'arbre phylogénétique (substitution Kimura, option UPGMA) ont été réalisés avec le logiciel GCG (Genetics Computer Group, Madison WI).

### II - Résultats.

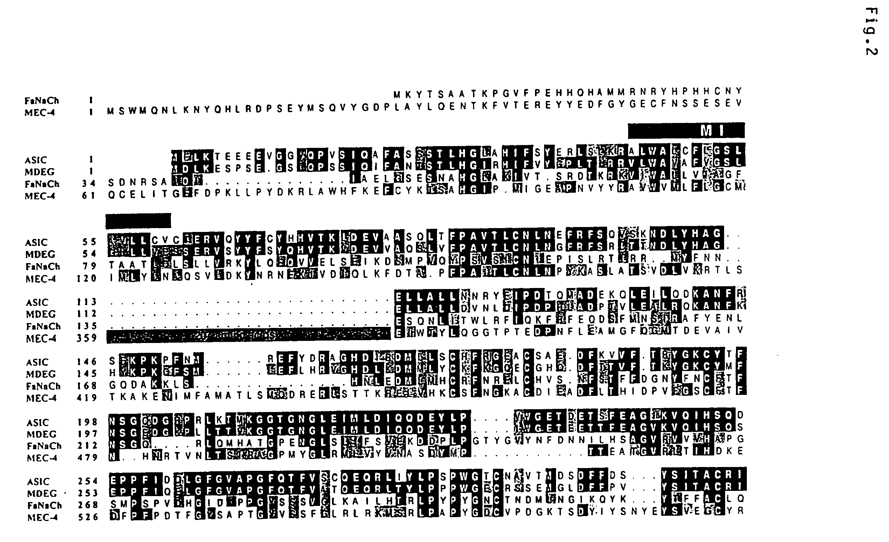

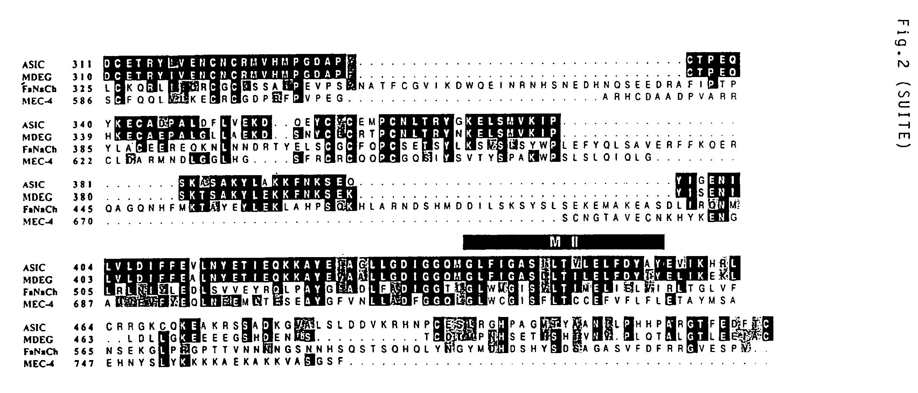

L'ADNc de 35 kb isolé de cerveau de rat code pour une protéine de 526 acides aminés qui présente, comme montré sur la figure 2, des homologies avec tous les membres clonés de la famille des canaux sodium de dégénérine sensibles à l'amiloride.

Comme montré sur la figure 5, l'expression de l'ARNc dans des oocytes de Xenopus a induit un courant entrant activé par les protons H⁺. Les propriétés biophysiques et la pharmacologie du canal ASIC sont proches de celles décrites pour les canaux cationiques activés par les protons des neurones sensoriels (3, 15, 16). Une baisse du pH extracellulaire au dessous d'un pH de 6,9 active un courant entrant rapidement élévé et désensibilisé (figure 5 a et b). Ce canal est activé par les protons extracellulaires, puisque, comme montré sur la figure 5 (c et d), l'application d'un acide sur la face extracellulaire de patch outside-out active le canal. L'acidification intracellulaire d'oocytes et l'acidification de la face intracellulaire de patch outside-out n'active pas le canal ASIC ni n'altère le courant ASIC induit par les protons extracellulaires.

L'analyse des courbes I-V de la figure 5 (c et e) enregistrées avec différents cations extracellulaires montre que Na⁺ est l'ion perméable majoritaire (canal de conductance simple 14,3 pS). Comme le canal ionique sensible aux protons des neurones sensoriels (15, 16), le canal ASIC discrimine faiblement entre les cations (figure 5 c, e, f). En effet, le canal est aussi perméable à Li⁺, K⁺, Ca²⁺ et H⁺, avec des rapports pNa⁺/pLi⁺ = 1,3 (figure 5 c, e), pNa⁺/pK⁺ = 13 (figure 5 c, e), pNa⁺/Ca²⁺ = 2,5 (figure 5 e) et pNa⁺/H⁺ = 0,8 (figure 5 f). La perméabilité au Ca²⁺ de ASIC pourrait être un chemin d'entrée voltage indépendant de Ca²⁺ dans la cellule. Un courant entrant de Ca²⁺ dans la cellule à travers les canaux ASIC peut être détecté en l'absence de Na⁺ extracellulaire (figure 6 a, b). Comme indiqué sur la figure 5 (e) la conductance unitaire pour Ca²⁺ était de 5,2 pS. En présence de 140 mM de Na⁺ extracellulaire, l'augmentation des concentrations en Ca²⁺ externe, a diminué l'amplitude du courant activé par les protons (figure 6c), démontrant ainsi que Ca²⁺ inhibe la perméabilité au Na⁺. Un blocage par le Ca²⁺ externe est caractéristique du I(H⁺) des neurones sensoriels (17).Le courant entrant activé par H⁺ dans les neurones sensoriels est inhibé par l'amiloride (18) et l'éthylisopropylamiloride (EIPA) (19). Comme montré à la figure 6 (d, e) le canal ASIC présente la même pharmacologie et est bloqué de façon réversible (Kd = 10 µM) par l'amiloride et ses dérivés benzamil et EIPA.

Par ailleurs, la protéine du canal ASIC présente environ 67% d'homologie de séquences avec le canal ionique de dégénérine dénommé "MDEG" (14) ou "BNaCI" (20). Toutefois, les propriétés électrophysiologiques de ces deux clones exprimés dans les oocytes de Xenopus sont clairement différentes :
- Comme montré sur la figure 5a, le canal MDEG n'est pas activé par les mêmes changements de pH que le canal ASIC.
- La substitution du résidu glycine en position 430 de MDEG par un acide aminé encombrant acide, comme la valine ou la phénylalanine active le canal (14), tout comme la mutation de l'alanine en position 704 de la dégénérine MEC-4 cause une neurodégéréscence chez *C. elegans* (12). Des mutations identiques d'ASIC (glycine en position 431 remplacée par la valine ou la phénylalanine) n'entraînent pas d'activité et les mutants ne peuvent pas être activés par les protons.

Les canaux cationiques activés par les protons ont été décrits dans les neurones sensoriels mais aussi dans les neurones du système nerveux central (21). La distribution tissulaire de l'expression de l'ARNm du canal ASIC est en accord avec cette observation. Comme rapporté dans la figure 7a, un transcrit de 4,3 kb a été détecté dans le cerveau par analyse en Northern blot, et les résultats de la RT-PCR rapportés à la figure 7b montrent que le ganglion de la racine dorsale exprime l'ARNm de ASIC. La figure 8 (a,b) montre que l'ARNm de ASIC est bien exprimé dans les petits neurones du ganglion de la racine dorsale, ce qui supporte le fait que ASIC est le canal cationique activé par les protons rapidement désensibilisant décrit dans les neurones sensoriels nociceptifs. Alors que la présence de canaux cationiques activés par les protons dans le ganglion de la racine dorsale est en accord avec leur fonction de détecteur de l'acidité dans la nociception, leur rôle dans le cerveau reste à établir. Les résultats d'hybridation *in situ* de la figure 8c montrent une expression large et hétérogène de l'ARNm du canal ASIC. Les niveaux d'expression les plus élevés ont été observés dans le bulbe olfactif principal, le cortex cérébral, l'hyppocampe, l'habenula, le noyau amygdaloïde basolatéral et le cerebellum. L'activité synaptique s'accompagne de changements du pH extracellulaire (22, 23) et les changements localisés rapides de pH dans ou à proximité de la fente synaptique sont sensiblement plus saturés et forts que les fluctuations macroscopiques du pH rapportées.

Les canaux cationiques activés par les protons sont les seuls canaux ioniques connus qui sont directement activés par un changement du pH et il a été envisagé que les fluctuations extracellulaires du pH jouent un rôle neuromodulateur (23). L'expression de canaux cationiques dans le cerveau supporte en outre l'hypothèse que les fluctuations de pH ne sont pas seulement une activation neuronale par un produit, mais davantage un chemin de communication dans le système nerveux central.

Outre les canaux cationiques activés par les protons rapidement inactivés, il a été rapporté la présence dans les neurones sensoriels de canaux cationiques activés par les protons présentant des cinétiques plus lentes (4, 24). Les canaux cationiques activés par les protons forment probablement, comme d'autre canaux cationiques activés par un ligand (25, 26), une famille de canaux cationiques où différentes sous-unités ou combinaisons de sous-unités constituent les canaux avec diverses propriétés pharmacologiques et biophysiques.

La sensation de l'acidité n'est pas uniquement impliquée dans la nociception, mais est aussi associée à la transduction du goût (2). Les stimulations acides activent les canaux cationiques activés par les protons dans les cellules du goût (2, 27) et l'amiloride inhibe la perception du goût acide (2). Aussi, les données tant physiologiques que pharmacologiques indiquent que ASIC et d'autres membres de cette famille sont impliqués dans la transduction du goût. Il est en effet particulièrement frappant que la même classe de canaux ioniques soit associée à différentes facettes de la perception sensorielle :
- Les canaux sodium sensibles à l'amiloride sont associés à la transduction du goût salé (2).
- Les dégénérines de *C. elegans* sont impliquées dans la mécanotransduction et ont été proposées comme formant des canaux ioniques mécanosensibles (28, 29).
- les canaux ASIC sont impliqués dans la nociception et dans la transduction du goût acide.

Le clonage du canal ASIC permet de disposer d'un nouvel outil d'investigation de ce groupe de canaux ioniques et de développer des bloquants spécifiques trouvant leur utilisation notamment comme analgésiques.

### Liste des références

1. Rang, H.P., Bevan, S. & Dray, A. Br. Med. Bull. 47, 534-548 (1991).
2. Lindemann, B. Physiol. Rev. 76, 718-766 (1996).
3. Krishtal, O.A. & Pidoplichko, V.I. Neuroscience 6, 2599-2601 (1981).
4. Bevan, S. & Geppetti, P. Trends Neurosci. 17, 509-512 (1994).
5. Akaike, N., Krishtal, O.A. & Maruyama, T. J. Neurophysiol. 63, 805-813 (1990).
6. Canessa, C.M., Horisberger, J.D. & Rossier, B.C. Nature 361, 467-470 (1993).
7. Canessa, C.M., Schild, L., Buell, G., Thorens, B., Gautschi, I., Horisberger, J.D. & Rossier, B.C. Nature 367, 463-467 (1994).
8. Lingueglia, E., Voilley, N., Waldmann, R., Lazdunski, M. & Barbry, P. Febs Lett. 318, 95-99 (1993).
9. Lingueglia, E., Renard, S., Waldmann, R., Voilley, N., Champigny, G., Plass, H., Lazdunski, M. & Barbry, P. J. Biol. Chem. 269, 13736-13739 (1994).
10. Lingueglia, E., Champigny, G., Lazdunski, M. & Barbry, P. Nature 378, 730-733 (1995).
11. Waldmann, R., Champigny, G., Bassilana, F., Voilley, N. & Lazdunski, M. J. Biol. Chem. 270, 27411-27414 (1995).
12. Driscoll, M. & Chalfie, M. Nature 349, 588-593 (1991).
13. Huang, M. & Chalfie, M. Nature 367, 467-470 (1994).
14. Waldmann, R, Champigny, G., Voilley, N., Lauritzen, I. & Lazdunski, M. J. Biol. Chem. 271, 10433-10434 (1996).
15. Kovalchuk Yu, N., Krishtal, O.A. & Nowycky, M.C. Neurosci. Lett. 115, 237-242 (1990).
16. Konnerth, A., Lux, H.D. & Morad, M. J. Physiol. 386, 603-633 (1987).
17. Davies, N.W., Lux, H.D. & Morad, M. J. Physiol. 400, 159-187 (1988).
18. Korkushko, A. O. & Krishtal, O.A. Neirofiziologiia 16, 557-561 (1984).
19. Grantyn, R, Perouansky, M., Rodriguez-Tebar, A. & Lux, H.D. Dev. Brain Res. 49, 150-155 (1989).
20. Price, M.P., Snyder, P.M. & Welsh, M.J. J. Biol. Chem. 271, 7879-7882 (1996).
21. Akaike, N. & Ueno, S. Prog. Neurobiol. 43, 73-83 (1994).
22. Krishtal, O.A., Osipchuk, Y.V., Shelest, T.N. & Smirnoff, S.V. Brain Res. 436, 352-356 (1987).
23. Chesler, M. & Kaila, K. Trends Neurosci. 15, 396-402 (1992).
24. Bevan, S. & Yeats, J. J. Physiol. 433, 145-161 (1991).
25. Lewis, C., Neidhart, S., Holy, C., North, R.A., Buell, G. & Surprenant, A. Nature 377, 432-435 (1995).
26. Barnard, E.A. Trends Pharmacol. Sci. 17, 305 - 309 (1996).
27. Okada, Y., Miyamoto, T. & Sato, T. J. Exp. Biol. 187, 19-32 (1994).
28. Liu, J., Schrank, B. & Waterston, R. Science 273, 361 (1996).
29. Waldmann, R., Champigny, G. & Lazdunski, M. J. Biol. Chem. 270, 11735-11737 (1995).
30. Renard, S., Lingueglia, E., Voilley, N., Lazdunski, M. & Barbry, P. J. Biol. Chem. 269, 12981-12986 (1994).

### LISTE DE SÉQUENCES.

### NOMBRE DE SÉQUENCES : 6

INFORMATION CONCERNANT LA SEO ID NO:1 :
   i) CARACTRERISTIQUE DE LA SEQUENCE :
      A) LONGUEUR: 3562 paires de base
      B) TYPE : acide nucléique
      C) NOMBRE DE BRINS: double
      D) CONFIGURATION: linéaire
   ii) TYPE DE MOLECULE: ADN
   vi) ORIGINE: rat
   ix) CARACTERISTIQUE
      A) NOM/CLE : ASIC
      B) LOCALISATION : 123 .. 1700
   xi) DESCRIPTION DE LA SEQUENCE : SEQ ID NO:1 : 
CACACACACA CACACACACA CACACACACA CACACACACA CACACAGAAC CTGCGCCTGT 60

GCCTGTGCCT GTGCCTGTGC CTGTTTGAGA GCTGGAGACA CAGAAGGATC CCCTTGGCAA 120

GG ATG GAA TTG AAG ACC GAG GAG GAG GAG GTG GGT GGT GTC CAG CCG 167
Met Glu Leu Lys Thr Glu Glu Glu Glu Val Gly Gly Val Gln Pro
1               5                   10                  15

GTG AGC ATC CAG GCT TTC GCC AGC AGC TCC ACG CTG CAT GGT CTT GCC 215
Val Ser Ile Gln Ala Phe Ala Ser Ser Ser Thr Leu His Gly Leu Ala
                20                  25                  30

CAC ATC TTC TCC TAT GAG CGG CTG TCT CTG AAG CGG GCA CTG TGG GCC 263
His Ile Phe Ser Tyr Glu Arg Leu Ser Leu Lys Arg Ala Leu Trp Ala
            35                  40                  45

CTG TGC TTC CTG GGT TCG CTG GCC GTC CTG CTG TGT GTG TGC ACT GAG 311
Leu Cys Phe Leu Gly Ser Leu Ala Val Leu Leu Cys Val Cys Thr Glu
        50                  55                  60

CGT GTG CAG TAC TAC TTC TGC TAT CAC CAC GTC ACC AAG CTT GAC GAA 359
Arg Val Gln Tyr Tyr Phe Cys Tyr His His Val Thr Lys Leu Asp Glu
    65                  70                  75

GTG GCT GCC TCC CAG CTC ACC TTC CCT GCT GTC ACA CTG TGC AAT CTC 407
Val Ala Ala Ser Gln Leu Thr Phe Pro Ala Val Thr Leu Cys Asn Leu
80                  85                  90                  95

AAT GAG TTC CGC TTT AGC CAA GTC TCC AAG AAT GAC CTG TAC CAT GCT 455
Asn Glu Phe Arg Phe Ser Gln Val Ser Lys Asn Asp Leu Tyr His Ala
                100                 105                 110

GGG GAG CTG CTG GCC CTG CTC AAC AAC AGG TAT GAG ATC CCG GAC ACA 503
Gly Glu Leu Leu Ala Leu Leu Asn Asn Arg Tyr Glu Ile Pro Asp Thr
            115                 120                 125
 
CAG ATG GCT GAT GAA AAG CAG CTA GAG ATA TTG CAG GAC AAG GCC AAC 551
Gln Met Ala Asp Glu Lys Gln Leu Glu Ile Leu Gln Asp Lys Ala Asn
        130                 135                 140

TTC CGG AGC TTC AAG CCC AAG CCC TTC AAC ATG CGT GAA TTC TAC GAC 599
Phe Arg Ser Phe Lys Pro Lys Pro Phe Asn Met Arg Glu Phe Tyr Asp
    145                 150                 155

AGA GCG GGG CAC GAT ATT CGA GAC ATG CTG CTC TCG TGC CAC TTC CGT 647
Arg Ala Gly His Asp Ile Arg Asp Met Leu Leu Ser Cys His Phe Arg
160                 165                 170                 175

GGG GAG GCC TGC AGC GCT GAA GAT TTC AAA GTG GTC TTC ACT CGG TAT 695
Gly Glu Ala Cys Ser Ala Glu Asp Phe Lys Val Val Phe Thr Arg Tyr
                180                 185                 190

GGG AAG TGT TAC ACA TTC AAC TCG GGC CAA GAT GGG CGG CCA CGG CTG 743
Gly Lys Cys Tyr Thr Phe Asn Ser Gly Gln Asp Gly Arg Pro Arg Leu
            195                 200                 205

AAG ACC ATG AAA GGT GGG ACT GGC AAT GGC CTG GAG ATC ATG CTG GAC 791
Lys Thr Met Lys Gly Gly Thr Gly Asn Gly Leu Glu Ile Met Leu Asp
        210                 215                 220

ATT CAG CAA GAT GAA TAT TTG CCT GTG TGG GGA GAG ACC GAC GAG ACA 839
Ile Gln Gln Asp Glu Tyr Leu Pro Val Trp Gly Glu Thr Asp Glu Thr
    225                 230                 235

TCC TTC GAA GCA GGC ATC AAA GTG CAG ATC CAC AGT CAG GAT GAA CCC 887
Ser Phe Glu Ala Gly Ile Lys Val Gln Ile His Ser Gln Asp Glu Pro
240                 245                 250                 255

CCT TTC ATC GAC CAG CTG GGC TTT GGT GTG GCT CCA GGT TTC CAG ACG 935
Pro Phe Ile Asp Gln Leu Gly Phe Gly Val Ala Pro Gly Phe Gln Thr
                260                 265                 270

TTT GTG TCT TGC CAG GAG CAG AGG CTC ATC TAC CTG CCC TCA CCC TGG 983
Phe Val Ser Cys Gln Glu Gln Arg Leu Ile Tyr Leu Pro Ser Pro Trp
            275                 280                 285

GGC ACC TGC AAT GCT GTT ACC ATG GAC TCG GAT TTC TTC GAC TCC TAC 1031
Gly Thr Cys Asn Ala Val Thr Met Asp Ser Asp Phe Phe Asp Ser Tyr
        290                 295                 300

AGC ATC ACT GCC TGC CGG ATT GAT TGC GAG ACG CGT TAC CTG GTG GAG 1079
Ser Ile Thr Ala Cys Arg Ile Asp Cys Glu Thr Arg Tyr Leu Val Glu
    305                 310                 315

AAC TGC AAC TGC CGT ATG GTG CAC ATG CCA GGG GAC GCC CCA TAC TGC 1127
Asn Cys Asn Cys Arg Met Val His Met Pro Gly Asp Ala Pro Tyr Cys
320                 325                 330                 335

ACT CCA GAG CAG TAC AAG GAG TGT GCA GAT CCT GCC CTG GAC TTC CTA 1175
Thr Pro Glu Gln Tyr Lys Glu Cys Ala Asp Pro Ala Leu Asp Phe Leu
                340                 345                 350

GTG GAG AAA GAC CAG GAA TAC TGC GTG TGT GAG ATG CCT TGC AAC CTG 1223
Val Glu Lys Asp Gln Glu Tyr Cys Val Cys Glu Met Pro Cys Asn Leu
            355                 360                 365
 
ACC CGC TAC GGC AAG GAG CTG TCC ATG GTC AAG ATC CCA AGC AAA GCC 1271
Thr Arg Tyr Gly Lys Glu Leu Ser Met Val Lys Ile Pro Ser Lys Ala
        370                 375                 380

TCC GCC AAG TAC CTG GCC AAG AAG TTC AAC AAA TCG GAG CAG TAC ATA 1319
Ser Ala Lys Tyr Leu Ala Lys Lys Phe Asn Lys Ser Glu Gln Tyr Ile
    385                 390                 395

GGG GAG AAC ATT CTG GTG CTG GAC ATT TTC TTT GAA GTC CTC AAC TAT 1367
Gly Glu Asn Ile Leu Val Leu Asp Ile Phe Phe Glu Val Leu Asn Tyr
400                 405                 410                 415

GAG ACC ATC GAG CAG AAA AAG GCC TAT GAG ATC GCA GGG CTG TTG GGT 1415
Glu Thr Ile Glu Gln Lys Lys Ala Tyr Glu Ile Ala Gly Leu Leu Gly
                420                 425                 430

GAC ATC GGG GGC CAG ATG GGG TTG TTC ATC GGT GCC AGC ATC CTC ACC 1463
Asp Ile Gly Gly Gln Met Gly Leu Phe Ile Gly Ala Ser Ile Leu Thr
            435                 440                 445

GTG CTG GAA CTC TTT GAC TAT GCC TAC GAG GTC ATT AAG CAC AGG CTG 1511
Val Leu Glu Leu Phe Asp Tyr Ala Tyr Glu Val Ile Lys His Arg Leu
        450                 455                 460

TGC AGA CGT GGA AAG TGC CAG AAG GAG GCT AAG AGG AGC AGC GCA GAC 1559
Cys Arg Arg Gly Lys Cys Gln Lys Glu Ala Lys Arg Ser Ser Ala Asp
    465                 470                 475

AAG GGC GTG GCG CTC AGC CTG GAT GAC GTC AAA AGA CAC AAT CCC TGC 1607
Lys Gly Val Ala Leu Ser Leu Asp Asp Val Lys Arg His Asn Pro Cys
480                 485                 490                 495

GAG AGC CTC CGA GGA CAT CCT GCC GGG ATG ACG TAC GCT GCC AAC ATC 1655
Glu Ser Leu Arg Gly His Pro Ala Gly Met Thr Tyr Ala Ala Asn Ile
                500                 505                 510

CTA CCT CAC CAT CCC GCT CGA GGC ACG TTT GAG GAC TTT ACC TGC TAA 1703
Leu Pro His His Pro Ala Arg Gly Thr Phe Glu Asp Phe Thr Cys *
515 520 526

GCCCTCGCAG GCCGCTGTAC CAAAGGCCTA GGTGGGGAGG GCTGGGGGAG CAAGGGGCCC 1763

CCAACTGCCC CCAGCTACCC TGTGGACTTA ACTGCATTCC TGGTCAGTGG TTCCCTCTTG 1823

TCTGTGGTGA GAAAGGAGTC TTGACCATAG AGTCCTCTCC CAGCCTCTAT CCCATCTTTT 1943

TATTTTAATT TAATCACATT TGCTCTGTAA TATTGCTTGA GGCTGGGGAT CGTGATTTCC 2003

CCCCAGTTCT TTTATTGTTG AGAATAGTTT TCTCTATTCT GGGTTTTCTG TTATTTCAAA 2063
TGAATCTGCA AATTGCTCTT CCCATCTCTA TGAAGAATTG CGTTGGAATT TTGATGGGGA 2123

TTGTATTGAA TCTGTAGATT GCCTTTGGTA AGATGGCCAT TTTTACTATG TTAATCCTGC 2183

CAATTCATGA GCAAGGGAGA TCTTTCTATC TCTGAAATCT ACTTCAGTTT CTTTCTTCAG 2243

AGACTTGAAG TTCTTGTCAT AAAAATCTTT TTGGTTAGAG CCACACCAAG GTATTTTATA 2303

TTGTTTGTGA CTATTGTGAA TGGTGTCATT TCCCTAATTT CCTTCTCAGC CTACTTATCC 2363
 
TTTGAGTAGA GGAAGGCTTC TGATTTGTTT GGGTTAATTT TATACCCAGC TGCTTTGCTA 2423

AAGTTCTTTA TCAGGTTTAG GTGTTCTCTG GTGGAACTTT TGGGGTCACG TAAGAATACT 2483

ATTATATCAT CTGCAAATAG TGATATTTCA CTTCTTCCTT TCCAATTTCT ATCCCTCTGG 2543

GGACTTTTTG TTGTCTAATT GCTCTGGCTA GGACTTCAAA TTCTATATTG AATAGATAGG 2603

GAGAGAGTGG GCAGCCTTGT CTAGTTCCTG GTTTTCGTGG GATCGCTTCA AATTTCTCTC 2663

CATTTAGTTT GATATTGGCT ACTGGTTTGC TGTATATGGC TTTTACTGTA CTTAGGTATG 2723

GGCCTTGAAT TCCTGATATT TCCAAGACTT TTAACATGAA GGGGTTTTGA AATTTGCCAA 2783

ATGCTTTCTC AGCATCTAAT GAGATGATCA TGTGCCCTCC CCCCACCTTG AGTTTGTTTA 2843

TATAGTGGGT TACATGAAAG GATCATTTCT AATAGTCCAC AAGTCTGCCA AATCTTGCTG 2903

ATTGTGACTC ATTTCCATAG CAGGCTCTAT AACTTCTCTA ACAGATTGCA TTAAACTCTG 2963

CTTGGGGAAG GCATTACCTC TTGGTTGAAG CAATGTTGTA GTTTCTATGC CTGCTGAGTA 3023

AATAGCCTCA AGTCCAAGTA CTTGCCCAGA CTAATGATCA AACGTATCCA GGAGTTCCAT 3083

ACCAGAGATG TACTCTTCTC TCCTTTGAAG TACATTGCTG GAAGAGTAAT TGTGTTTGCT 3143

AGAGATACTC CTTCGAACTG CAAAAGAAAT CTCTTGGCTA AGCATATAAT CAAGCCTCAG 3203

GTTTTCTTTT TATTAAATAG CTGCTTGTAA GAAAGTGGAC ACTAAGCATA TACCTCAAAG 3263

GGAGACAGAA TGACTCTGTG CCTTCACTGA TGGAAGTCTG GGTTACAAAT TACATCAGAA 3323

GAACCTATCA TAGTGAAACA TCTCATTCCC CTGGTATAAT CCCTTCTAGA AATACACTTG 3383

TGACTCTGAA ATGTTATAAT CGTGACAACT AGGCTGTTAC AGATACACCA AGTTAAATTT 3443

GATAGAGAAA CCAGGCTTGG AGCCTCATGT CCATAGGGCA AGAGGAAGAT GCTGAGTGTT 3503

TAAGGTTGGT TTGAGCGAAG AACAATACCT TGTGTCACAA AAATGAAAGG AAAAAAGAAA 3563

AAAGGAAAGA AGGAAAGAAA GAGAGAGAAA GAAAAAGAAA GAAAGAAAAA AAAAAAAAA 3562
INFORMATION CONCERNANT LA SEQ ID NO:2 :
   i) CARACTRERISTIQUE DE LA SEQUENCE:
      A) LONGUEUR : 1620 paires de base
      B) TYPE : acide nucléique
      C) NOMBRE DE BRINS: double
      D) CONFIGURATION : linéaire
   ii) TYPE DE MOLECULE : ADN
   vi) ORIGINE : homme
   ix) CARACTERISTIQUE
      A) NOM/CLE : ASIC
      B) LOCALISATION : 1 .. 1542
   xi) DESCRIPTION DE LA SEQUENCE: SEQ ID NO:2 : 
CCG GTG AGC ATC CAG GCC TTC GCC AGC AGC TCC ACA CTG CAC GGC ATG 48
Pro Val Ser Ile Gln Ala Phe Ala Ser Ser Ser Thr Leu His Gly Met
1               5                   10                  15

GCC CAC ATC TTC TCC TAC GAG CGG CTG TCT CTG AAG CGG GCA CTG TGG 96
Ala His Ile Phe Ser Tyr Glu Arg Leu Ser Leu Lys Arg Ala Leu Trp
            20                  25                  30

GCC CTG TGC TTC CTG GGC TCG CTG GCT GTG CTG CTG TGT GTG TGC ACG 144
Ala Leu Cys Phe Leu Gly Ser Leu Ala Val Leu Leu Cys Val Cys Thr
        35                  40                  45

GAG CGT GTG CAG TAC TAC TTC CAC TAc CAC CAT GTC ACC AAG CTC GAC 192
Glu Arg Val Gln Tyr Tyr Phe His Tyr His His Val Thr Lys Leu Asp
    50                  55                  60

GAG GTG GCT GCC TCT CAG CTT ACC TTC CCT GCT GTC ACG CTG TGC AAC 240
Glu Val Ala Ala Ser Gln Leu Thr Phe Pro Ala Val Thr Leu Cys Asn
65                  70                  75                  80

CTC AAC GAG TTC CGC TTT AGC CAA GTC TCC AAG AAT GAC CTG TAT CAT 288
Leu Asn Glu Phe Arg Phe Ser Gln Val Ser Lys Asn Asp Leu Tyr His
                85                  90                  95

GCT GGG GAG CTG CTG GCC CTG CTC AAC AAC AGG TAT GAG ATA CCA GAC 336
Ala Gly Glu Leu Leu Ala Leu Leu Asn Asn Arg Tyr Glu Ile Pro Asp
            100                 105                 110

ACA CAG ATG GCA GAT GAA AAG CAG CTG GAG ATA CTG CAG GAC AAA GCC 384
Thr Gln Met Ala Asp Glu Lys Gln Leu Glu Ile Leu Gln Asp Lys Ala
        115                 120                 125

AAC TTC CGC AGC TTC AAA CCC AAA CCC TTC AAC ATG CGT GAG TTC TAC 432
Asn Phe Arg Ser Phe Lys Pro Lys Pro Phe Asn Met Arg Glu Phe Tyr
    130                 135                 140

GAC CGA GCT GGG CAC GAC ATT CGA GAC ATG CTG CTC TCC TGC CAC TTC 480
Asp Arg Ala Gly His Asp Ile Arg Asp Met Leu Leu Ser Cys His Phe
145                 150                 155                 160
 
CGG GGG GAG GTC TGC AGC GCT GAA GAC TTC AAG GTG GTC TTC ACA CGC 528
Arg Gly Glu Val Cys Ser Ala Glu Asp Phe Lys Val Val Phe Thr Arg
                165                 170                 175

TAT GGA AAG TGC TAC ACG TTC AAC TCG GGC CGA AAT GGG CGG CCG CGG 576
Tyr Gly Lys Cys Tyr Thr Phe Asn Ser Gly Arg Asn Gly Arg Pro Arg
            180                 185                 190

CTG AAG ACC ATG AAG GGT GGG ACG GGC AAT GGG CTG GAA ATC ATG CTG 624
Leu Lys Thr Met Lys Gly Gly Thr Gly Asn Gly Leu Glu Ile Met Leu
        195                 200                 205

GAC ATC CAG CAG GAC GAG TAC CTG CCT GTG TGG GGG GAG ACT GAC GAG 672
Asp Ile Gln Gln Asp Glu Tyr Leu Pro Val Trp Gly Glu Thr Asp Glu
    210                 215                 220

ACG TCT TTC GAA GCA GGC ATC AAA GTG CAG ATC CAT AGT CAG GAT GAA 720
Thr Ser Phe Glu Ala Gly Ile Lys Val Gln Ile His Ser Gln Asp Glu
225                 230                 235                 240

CCT CCT TTC ATC GAC CAG CTG GGC TTT GGC GTG GCC CCA GGC TTC CAG 768
Pro Pro Phe Ile Asp Gln Leu Gly Phe Gly Val Ala Pro Gly Phe Gln
                245                 250                 255

ACC TTT GTG GCC TGC CAG GAG CAG CGG CTC ATA TAC CTG CCC CCA CCC 816
Thr Phe Val Ala Cys Gln Glu Gln Arg Leu Ile Tyr Leu Pro Pro Pro
            260                 265                 270

TGG GGC ACC TGC AAA GCT GTT ACC ATG GAC TCG GAT TTG GAT TTC TTC 864
Trp Gly Thr Cys Lys Ala Val Thr Met Asp Ser Asp Leu Asp Phe Phe
        275                 280                 285

GAC TCC TAC AGC ATC ACT GCC TGC CGC ATC GAC TGT GAG ACG CGC TAC 912
Asp Ser Tyr Ser Ile Thr Ala Cys Arg Ile Asp Cys Glu Thr Arg Tyr
    290                 295                 300

CTG GTG GAG AAC TGC AAC TGC CGC ATG GTG CAC ATG CCA GGG GAT GCC 960
Leu Val Glu Asn Cys Asn Cys Arg Met Val His Met Pro Gly Asp Ala
305                 310                 315                 320

CCA TAC TGT ACT CCA GAG CAG TAC AAG GAG TGT GCA GAT CCT GCT CTG 1008
Pro Tyr Cys Thr Pro Glu Gln Tyr Lys Glu Cys Ala Asp Pro Ala Leu
                325                 330                 335

GAC TTC CTG GTG GAG AAG GAC CAG GAG TAC TGC GTG TGT GAA ATG CCT 1056
Asp Phe Leu Val Glu Lys Asp Gln Glu Tyr Cys Val Cys Glu Met Pro
            340                 345                 350

TGC AAC CTG ACC CGC TAT GGC AAA GAG CTG TCC ATG GTC AAG ATC CCC 1104
Cys Asn Leu Thr Arg Tyr Gly Lys Glu Leu Ser Met Val Lys Ile Pro
        355                 360                 365

AGC AAA GCC TCA GCC AAG TAC CTG GCC AAG AAG TTC AAC AAA TCT GAG 1152
Ser Lys Ala Ser Ala Lys Tyr Leu Ala Lys Lys Phe Asn Lys Ser Glu
    370                 375                 380

CAA TAC ATA GGG GAG AAC ATC CTG GTG CTG GAC ATT TTC TTT GAA GTC 1200
Gln Tyr Ile Gly Glu Asn Ile Leu Val Leu Asp Ile Phe Phe Glu Val
385                 390                 395                 400
 
CTC AAC TAT GAG ACC ATT GAA CAG AAG AAG GCC TAT GAG ATT GCA GGG 1248
Leu Asn Tyr Glu Thr Ile Glu Gln Lys Lys Ala Tyr Glu Ile Ala Gly
                405                 410                 415

CTC CTG GGT GAC ATC GGG GGC CAG ATG GGG CTG TTC ATC GGG GCC AGC 1296
Leu Leu Gly Asp Ile Gly Gly Gln Met Gly Leu Phe Ile Gly Ala Ser
            420                 425                 430

ATC CTC ACG GTG CTG GAG CTC TTT GAC TAC GCC TAC GGG GTC ATT AAG 1344
Ile Leu Thr Val Leu Glu Leu Phe Asp Tyr Ala Tyr Gly Val Ile Lys
        435                 440                 445

CAC AAG CTG TGC CGA CGA GGA AAA TGC CAG AAG GAG GCC AAA AGG AGC 1392
His Lys Leu Cys Arg Arg Gly Lys Cys Gln Lys Glu Ala Lys Arg Ser
    450                 455                 460

AGT GCG GAC AAG GGC GTG GCC CTC AGC CTG GAC GAC GTC AAA AGA CAC 1440
Ser Ala Asp Lys Gly Val Ala Leu Ser Leu Asp Asp Val Lys Arg His
465                 470                 475                 480

AAC CCG TGC GAG AGC CTT CGG GGC CAC CCT GCC GGG ATG ACA TAC GCT 1488
Asn Pro Cys Glu Ser Leu Arg Gly His Pro Ala Gly Met Thr Tyr Ala
                485                 490                 495

GCC AAC ATC GTA CCT CAC CAT CCG GCC CGA GGC ACG TTC GAG GAC TTT 1536
Ala Asn Ile Val Pro His His Pro Ala Arg Gly Thr Phe Glu Asp Phe
            500                 505                 510

ACC TGC TGA GCCCCGCAGG CCGCCGAACC AAAGACCTAG ATGGGGAGGA CTAGGAGAGC 1595
Thr Cys *
514

GAGGGGGCCC CCAGCTGCCT CCTAA 1620
INFORMATION CONCERNANT LA SEQ ID NO:3 :
   i) CARACTRERISTIQUE DE LA SEQUENCE :
      A) LONGUEUR : 1666 paires de base
      B) TYPE : acide nucléique
      C) NOMBRE DE BRINS : double
      D) CONFIGURATION : linéaire
   ii) TYPE DE MOLECULE : ADN
   vi) ORIGINE : homme
   ix) CARACTERISTIQUE
      A) NOM/CLE : MDEG
      B) LOCALISATION : 127 .. 1663
   xi) DESCRIPTION DE LA SEQUENCE : SEQ ID NO:3 : 
TCTGGCGCGA TGCTTACCTT GCGTTCTCTC CCCTGAACGT CAAGGTTTAA GCAGAGCCCG 60

AGGACTGGGA GCTCTTCTCT GAAATTCGAT CAACCTGAAG CCAGTTGCGG AACTGCACGG 120

GGTCCCG ATG GAC CTC AAG GAA AGC CCC AGT GAG GGC AGC CTG CAA CCT 169
Met Asp Leu Lys Glu Ser Pro Ser Glu Gly Ser Leu Gln Pro
1               5                   10

TCT AGC ATC CAG ATC TTT GCC AAC ACC TCC ACC CTC CAT GGC ATC CGC 217
Ser Ser Ile Gln Ile Phe Ala Asn Thr Ser Thr Leu His Gly Ile Arg
15                  20                  25                  30

CAC ATC TTC GTG TAT GGG CCG CTG ACC ATC CGG CGT GTG CTG TGG GCA 265
His Ile Phe Val Tyr Gly Pro Leu Thr Ile Arg Arg Val Leu Trp Ala
                35                  40                  45

GTG GCC TTC GTG GGC TCT CTG GGC CTG CTG CTG GTG GAG AGC TCT GAG 313
Val Ala Phe Val Gly Ser Leu Gly Leu Leu Leu Val Glu Ser Ser Glu
            50                  55                  60

AGG GTG TCC TAC TAC TTC TCC TAC CAG CAT GTC ACT AAG GTG GAC GAA 361
Arg Val Ser Tyr Tyr Phe Ser Tyr Gln His Val Thr Lys Val Asp Glu
        65                  70                  75

GTG GTG GCT CAA AGC CTG GTC TTC CCA GCT GTG ACC CTC TGT AAC CTC 409
Val Val Ala Gln Ser Leu Val Phe Pro Ala Val Thr Leu Cys Asn Leu
    80                  85                  90

AAT GGC TTC CGG TTC TCC AGG CTC ACC ACC AAC GAC CTG TAC CAT GCT 457
Asn Gly Phe Arg Phe Ser Arg Leu Thr Thr Asn Asp Leu Tyr His Ala
95                  100                 105                 110

GGG GAG CTG CTG GCC CTG CTG GAT GTC AAC CTG CAG ATC CCG GAC CCC 505
Gly Glu Leu Leu Ala Leu Leu Asp Val Asn Leu Gln Ile Pro Asp Pro
                115                 120                 125

CAT CTG GCT GAC CCC TCC GTG CTG GAG GCC CTG CGG CAG AAG GCC AAC 553
His Leu Ala Asp Pro Ser Val Leu Glu Ala Leu Arg Gln Lys Ala Asn
            130                 135                 140
 
TTC AAG CAC TAC AAA CCC AAG CAG TTC AGC ATG CTG GAG TTC CTG CAC 601
Phe Lys His Tyr Lys Pro Lys Gln Phe Ser Met Leu Glu Phe Leu His
        145                 150                 155

CGT GTG GGC CAT GAC CTG AAG GAT ATG ATG CTC TAC TGC AAG TTC AAA 649
Arg Val Gly His Asp Leu Lys Asp Met Met Leu Tyr Cys Lys Phe Lys
    160                 165                 170

GGG CAG GAG TGC GGC CAC CAA GAC TTC ACC ACA GTG TTT ACA AAA TAT 697
Gly Gln Glu Cys Gly His Gln Asp Phe Thr Thr Val Phe Thr Lys Tyr
175                 180                 185                 190

GGG AAG TGT TAC ATG TTT AAC TCA GGC GAG GAT GGC AAA CCT CTG CTC 745
Gly Lys Cys Tyr Met Phe Asn Ser Gly Glu Asp Gly Lys Pro Leu Leu
                195                 200                 205

ACC ACG GTC AAG GGG GGG ACA GGC AAC GGG CTG GAG ATC ATG CTG GAC 793
Thr Thr Val Lys Gly Gly Thr Gly Asn Gly Leu Glu Ile Met Leu Asp
            210                 215                 220

ATT CAG CAG GAT GAG TAC CTG CCC ATC TGG GGA GAG ACA GAG GAA ACG 841
Ile Gln Gln Asp Glu Tyr Leu Pro Ile Trp Gly Glu Thr Glu Glu Thr
        225                 230                 235

ACA TTT GAA GCA GGA GTG AAA GTT CAG ATC CAC AGT CAG TCT GAG CCA 889
Thr Phe Glu Ala Gly Val Lys Val Gln Ile His Ser Gln Ser Glu Pro
    240                 245                 250

CCT TTC ATC CAA GAG CTG GGC TTT GGG GTG GCT CCA GGG TTC CAG ACC 937
Pro Phe Ile Gln Glu Leu Gly Phe Gly Val Ala Pro Gly Phe Gln Thr
255                 260                 265                 270

TTT GTG GCC ACA CAG GAG CAG AGG CTC ACA TAC CTG CCC CCA CCG TGG 985
Phe Val Ala Thr Gln Glu Gln Arg Leu Thr Tyr Leu Pro Pro Pro Trp
                275                 280                 285

GGT GAG TGC CGA TCC TCA GAG ATG GGC CTC GAC TTT TTT CCT GTT TAC 1033
Gly Glu Cys Arg Ser Ser Glu Met Gly Leu Asp Phe Phe Pro Val Tyr
            290                 295                 300

AGC ATC ACC GCC TGT AGG ATT GAC TGT GAG ACC CGC TAC ATT GTG GAA 1081
Ser Ile Thr Ala Cys Arg Ile Asp Cys Glu Thr Arg Tyr Ile Val Glu
        305                 310                 315

AAC TGC AAC TGC CGC ATG GTT CAC ATG CCA GGG GAT GCC CCT TTT TGT 1129
Asn Cys Asn Cys Arg Met Val His Met Pro Gly Asp Ala Pro Phe Cys
    320                 325                 330

ACC CCT GAG CAG CAC AAG GAG TGT GCA GAG CCT GCC CTA GGT CTG TTG 1177
Thr Pro Glu Gln His Lys Glu Cys Ala Glu Pro Ala Leu Gly Leu Leu
335                 340                 345                 350

GCG GAA AAG GAC AGC AAT TAC TGT CTC TGC AGG ACA CCC TGC AAC CTA 1225
Ala Glu Lys Asp Ser Asn Tyr Cys Leu Cys Arg Thr Pro Cys Asn Leu
                355                 360                 365

ACC CGC TAC AAC AAA GAG CTC TCC ATG GTG AAG ATC CCC AGC AAG ACA 1273
Thr Arg Tyr Asn Lys Glu Leu Ser Met Val Lys Ile Pro Ser Lys Thr
            370                 375                 380
 
TCA GCC AAG TAC CTT GAG AAG AAA TTT AAC AAA TCA GAA AAA TAT ATC 1321
Ser Ala Lys Tyr Leu Glu Lys Lys Phe Asn Lys Ser Glu Lys Tyr Ile
        385                 390                 395

TCA GAG AAC ATC CTT GTT CTG GAT ATA TTT TTT GAA GCT CTC AAT TAT 1369
Ser Glu Asn Ile Leu Val Leu Asp Ile Phe Phe Glu Ala Leu Asn Tyr
    400                 405                 410

GAG ACA ATT GAA CAG AAG AAG GCG TAT GAA GTT GCT GCC TTA CTT GGT 1417
Glu Thr Ile Glu Gln Lys Lys Ala Tyr Glu Val Ala Ala Leu Leu Gly
415                 420                 425                 430

GAT ATT GGT GGT CAG ATG GGA TTG TTC ATT GGT GCT AGT ATC CTT ACA 1465
Asp Ile Gly Gly Gln Met Gly Leu Phe Ile Gly Ala Ser Ile Leu Thr
                435                 440                 445

ATA CTA GAG CTC TTT GAT TAT ATT TAT GAG CTG ATC AAA GAG AAG CTA 1513
Ile Leu Glu Leu Phe Asp Tyr Ile Tyr Glu Leu Ile Lys Glu Lys Leu
            450                 455                 460

TTA GAC CTG CTT GGC AAA GAG GAG GAT GAA GGG AGC CAC GAT GAG AAT 1561
Leu Asp Leu Leu Gly Lys Glu Glu Asp Glu Gly Ser His Asp Glu Asn
        465                 470                 475

GTG AGT ACT TGT GAC ACA ATG CCA AAC CAC TCT GAA ACC ATC AGT CAC 1609
Val Ser Thr Cys Asp Thr Met Pro Asn His Ser Glu Thr Ile Ser His
    480                 485                 490

ACT GTG AAC GTG CCC CTG CAG ACG ACC CTG GGG ACC CTG GAA GAA ATA 1657
Thr Val Asn Val Pro Leu Gln Thr Thr Leu Gly Thr Leu Glu Glu Ile
495                 500                 505                 510

GCC TGC TGA 1666
Ala Cys *
512
INFORMATION CONCERNANT LA SEQ ID NO:4 :
   i) CARACTRERISTIQUE DE LA SEQUENCE :
      A) LONGUEUR : 3647 paires de base
      B) TYPE : acide nucléique
      C) NOMBRE DE BRINS : double
      D) CONFIGURATION : linéaire
   ii) TYPE DE MOLECULE : ADN
   vi) ORIGINE : rat
   ix) CARACTERISTIQUE
      A) NOM/CLE : ASIC1B
      B) LOCALISATION : 109 .. 1785
   xi) DESCRIPTION DE LA SEQUENCE : SEQ ID NO:4 : 
CTGCCACAGA GGCTCTGGTG AGGAAGGACA GACAGCTGGA CCGGCGCAGA CCTAGCCGAA 60

GTCCAACCTC CGTCCCTTCT GGTGGCTTCT TCCTGTCTCC TGAACAAG ATG CCC ATC 117
Met Pro Ile
1 3

CAG ATC TTT TGT TCT GTG TCA TTC TCC TCT GGA GAG GAG GCC CCG GGA 165
Gln Ile Phe Cys Ser Val Ser Phe Ser Ser Gly Glu Glu Ala Pro Gly
    5                   10                  15

TCC ATG GCA GAT ATC TGG GGT CCC CAC CAC CAC CGG CAG CAG CAG GAC 213
Ser Met Ala Asp Ile Trp Gly Pro His His His Arg Gln Gln Gln Asp
20                  25                  30                  35

AGC TCA GAA TCG GAA GAA GAG GAA GAG AAG GAA ATG GAG GCA GGG TCG 261
Ser Ser Glu Ser Glu Glu Glu Glu Glu Lys Glu Met Glu Ala Gly Ser
                40                  45                  50

GAG TTG GAT GAG GGT GAT GAC TCA CCT AGG GAC TTG GTG GCC TTC GCC 309
Glu Leu Asp Glu Gly Asp Asp Ser Pro Arg Asp Leu Val Ala Phe Ala
            55                  60                  65

AAC AGC TGT ACC TTC CAT GGT GCC AGC CAT GTG TTT GTG GAA GGG GGC 357
Asn Ser Cys Thr Phe His Gly Ala Ser His Val Phe Val Glu Gly Gly
        70                  75                  80

CCA GGG CCA AGG CAG GCC TTA TGG GCA GTG GCC TTT GTC ATA GCA CTG 405
Pro Gly Pro Arg Gln Ala Leu Trp Ala Val Ala Phe Val Ile Ala Leu
    85                  90                  95

GGT GCC TTC CTG TGC CAG GTA GGG GAC CGC GTT GCT TAT TAC CTC AGC 453
Gly Ala Phe Leu Cys Gln Val Gly Asp Arg Val Ala Tyr Tyr Leu Ser
100                 105                 110                 115

TAC CCA CAC GTG ACT TTG CTA GAC GAA GTG GCC ACC ACG GAG CTG GTC 501
Tyr Pro His Val Thr Leu Leu Asp Glu Val Ala Thr Thr Glu Leu Val
                120                 125                 130
 
TTC CCA GCT GTC ACC TTC TGC AAC ACC AAT GCC GTG CGG TTG TCC CAG 549
Phe Pro Ala Val Thr Phe Cys Asn Thr Asn Ala Val Arg Leu Ser Gln
            135                 140                 145

CTC AGC TAC CCT GAC TTG CTC TAC CTG GCC CCC ATG CTA GGA CTG GAT 597
Leu Ser Tyr Pro Asp Leu Leu Tyr Leu Ala Pro Met Leu Gly Leu Asp
        150                 155                 160

GAG AGT GAT GAC CCC GGG GTG CCC CTT GCT CCT CCT GGC CCA GAG GCT 645
Glu Ser Asp Asp Pro Gly Val Pro Leu Ala Pro Pro Gly Pro Glu Ala
    165                 170                 175

TTC TCC GGG GAG CCT TTT AAC CTC CAT CGT TTC TAT AAT CGC TCT TGC 693
Phe Ser Gly Glu Pro Phe Asn Leu His Arg Phe Tyr Asn Arg Ser Cys
180                 185                 190                 195

CAC CGG CTG GAG GAC ATG CTG CTC TAT TGT TCC TAC TGT GGG GGC CCC 741
His Arg Leu Glu Asp Met Leu Leu Tyr Cys Ser Tyr Cys Gly Gly Pro
                200                 205                 210

TGT GGT CCC CAC AAC TTC TCA GTG GTC TTC ACT CGG TAT GGG AAG TGT 789
Cys Gly Pro His Asn Phe Ser Val Val Phe Thr Arg Tyr Gly Lys Cys
            215                 220                 225

TAC ACA TTC AAC TCG GGC CAA GAT GGG CGG CCA CGG CTG AAG ACC ATG 837
Tyr Thr Phe Asn Ser Gly Gln Asp Gly Arg Pro Arg Leu Lys Thr Met
        230                 235                 240

AAA GGT GGG ACT GGC AAT GGC CTG GAG ATC ATG CTG GAC ATT CAG CAA 885
Lys Gly Gly Thr Gly Asn Gly Leu Glu Ile Met Leu Asp Ile Gln Gln
    245                 250                 255

GAT GAA TAT TTG CCT GTG TGG GGA GAG ACC GAC GAG ACA TCC TTC GAA 933
Asp Glu Tyr Leu Pro Val Trp Gly Glu Thr Asp Glu Thr Ser Phe Glu
260                 265                 270                 275

GCA GGC ATC AAA GTG CAG ATC CAC AGT CAG GAT GAA CCC CCT TTC ATC 981
Ala Gly Ile Lys Val Gln Ile His Ser Gln Asp Glu Pro Pro Phe Ile
                280                 285                 290

GAC CAG CTG GGC TTT GGT GTG GCT CCA GGT TTC CAG ACG TTT GTG TCT 1029
Asp Gln Leu Gly Phe Gly Val Ala Pro Gly Phe Gln Thr Phe Val Ser
            295                 300                 305

TGC CAG GAG CAG AGG CTC ATC TAC CTG CCC TCA CCC TGG GGC ACC TGC 1077
Cys Gln Glu Gln Arg Leu Ile Tyr Leu Pro Ser Pro Trp Gly Thr Cys
        310                 315                 320

AAT GCT GTT ACC ATG GAC TCG GAT TTC TTC GAC TCC TAC AGC ATC ACT 1125
Asn Ala Val Thr Met Asp Ser Asp Phe Phe Asp Ser Tyr Ser Ile Thr
    325                 330                 335

GCC TGC CGG ATT GAT TGC GAG ACG CGT TAC CTG GTG GAG AAC TGC AAC 1173
Ala Cys Arg Ile Asp Cys Glu Thr Arg Tyr Leu Val Glu Asn Cys Asn
340                 345                 350                 355
 
TGC CGT ATG GTG CAC ATG CCA GGG GAC GCC CCA TAC TGC ACT CCA GAG 1221
Cys Arg Met Val His Met Pro Gly Asp Ala Pro Tyr Cys Thr Pro Glu
                360                 365                 370

CAG TAC AAG GAG TGT GCA GAT CCT GCC CTG GAC TTC CTA GTG GAG AAA 1269
Gln Tyr Lys Glu Cys Ala Asp Pro Ala Leu Asp Phe Leu Val Glu Lys
            375                 380                 385

GAC CAG GAA TAC TGC GTG TGT GAG ATG CCT TGC AAC CTG ACC CGC TAC 1317
Asp Gln Glu Tyr Cys Val Cys Glu Met Pro Cys Asn Leu Thr Arg Tyr
        390                 395                 400

GGC AAG GAG CTG TCC ATG GTC AAG ATC CCA AGC AAA GCC TCC GCC AAG 1365
Gly Lys Glu Leu Ser Met Val Lys Ile Pro Ser Lys Ala Ser Ala Lys
    405                 410                 415

TAC CTG GCC AAG AAG TTC AAC AAA TCG GAG CAG TAC ATA GGG GAG AAC 1413
Tyr Leu Ala Lys Lys Phe Asn Lys Ser Glu Gln Tyr Ile Gly Glu Asn
420                 425                 430                 435

ATT CTG GTG CTG GAC ATT TTC TTT GAA GTC CTC AAC TAT GAG ACC ATC 1461
Ile Leu Val Leu Asp Ile Phe Phe Glu Val Leu Asn Tyr Glu Thr Ile
                440                 445                 450

GAG CAG AAA AAG GCC TAT GAG ATC GCA GGG CTG TTG GGT GAC ATC GGG 1509
Glu Gln Lys Lys Ala Tyr Glu Ile Ala Gly Leu Leu Gly Asp Ile Gly
            455                 460                 465

GGC CAG ATG GGG TTG TTC ATC GGT GCC AGC ATC CTC ACC GTG CTG GAA 1557
Gly Gln Met Gly Leu Phe Ile Gly Ala Ser Ile Leu Thr Val Leu Glu
        470                 475                 480

CTC TTT GAC TAT GCC TAC GAG GTC ATT AAG CAC AGG CTG TGC AGA CGT 1605
Leu Phe Asp Tyr Ala Tyr Glu Val Ile Lys His Arg Leu Cys Arg Arg
    485                 490                 495

GGA AAG TGC CAG AAG GAG GCT AAG AGG AGC AGC GCA GAC AAG GGC GTG 1653
Gly Lys Cys Gln Lys Glu Ala Lys Arg Ser Ser Ala Asp Lys Gly Val
500                 505                 510                 515

GCG CTC AGC CTG GAT GAC GTC AAA AGA CAC AAT CCC TGC GAG AGC CTC 1701
Ala Leu Ser Leu Asp Asp Val Lys Arg His Asn Pro Cys Glu Ser Leu
                520                 525                 530

CGA GGA CAT CCT GCC GGG ATG ACG TAC GCT GCC AAC ATC CTA CCT CAC 1749
Arg Gly His Pro Ala Gly Met Thr Tyr Ala Ala Asn Ile Leu Pro His
            535                 540                 545

CAT CCC GCT CGA GGC ACG TTT GAG GAC TTT ACC TGC TAA GCCCTCGCAG 1798
His Pro Ala Arg Gly Thr Phe Glu Asp Phe Thr Cys *
550 55 559

GCCGCTGTAC CAAAGGCCTA GGTGGGGAGG GCTGGGGGAG CAAGGGGCCC CCAACTGCCC 1858

CCAGCTACCC TGTGGACTTA ACTGCATTCC TGGTCAGTGG TTCCCTCTTG TCTGTGGTGA 1918

GAAAGGAGTC TTGACCATAG AGTCCTCTCC CAGCCTCTAT CCCATCTTTT TATTTTAATT 1978

TAATCACATT TGCTCTGTAA TATTGCTTGA GGCTGGGGAT CGTGATTTCC CCCCAGTTCT 2038
 
TTTATTGTTG AGAATAGTTT TCTCTATTCT GGGTTTTCTG TTATTTCAAA TGAATCTGCA 2098

AATTGCTCTT CCCATCTCTA TGAAGAATTG CGTTGGAATT TTGATGGGGA TTGTATTGAA 2158

TCTGTAGATT GCCTTTGGTA AGATGGCCAT TTTTACTATG TTAATCCTGC CAATTCATGA 2218

GCAAGGGAGA TCTTTCTATC TCTGAAATCT ACTTCAGTTT CTTTCTTCAG AGACTTGAAG 2278

TTCTTGTCAT AAAAATCTTT TTGGTTAGAG CCACACCAAG GTATTTTATA TTGTTTGTGA 2338

CTATTGTGAA TGGTGTCATT TCCCTAATTT CCTTCTCAGC CTACTTATCC TTTGAGTAGA 2398

GGAAGGCTTC TGATTTGTTT GGGTTAATTT TATACCCAGC TGCTTTGCTA AAGTTCTTTA 2458

TCAGGTTTAG GTGTTCTCTG GTGGAACTTT TGGGGTCACG TAAGAATACT ATTATATCAT 2518

CTGCAAATAG TGATATTTCA CTTCTTCCTT TCCAATTTCT ATCCCTCTGG GGACTTTTTG 2578

TTGTCTAATT GCTCTGGCTA GGACTTCAAA TTCTATATTG AATAGATAGG GAGAGAGTGG 2638

GCAGCCTTGT CTAGTTCCTG GTTTTCGTGG GATCGCTTCA AATTTCTCTC CATTTAGTTT 2698

GATATTGGCT ACTGGTTTGC TGTATATGGC TTTTACTGTA CTTAGGTATG GGCCTTGAAT 2758

TCCTGATATT TCCAAGACTT TTAACATGAA GGGGTTTTGA AATTTGCCAA ATGCTTTCTC 2818

AGCATCTAAT GAGATGATCA TGTGCCCTCC CCCCACCTTG AGTTTGTTTA TATAGTGGGT 2878

TACATGAAAG GATCATTTCT AATAGTCCAC AAGTCTGCCA AATCTTGCTG ATTGTGACTC 2938

ATTTCCATAG CAGGCTCTAT AACTTCTCTA ACAGATTGCA TTAAACTCTG CTTGGGGAAG 2998

GCATTACCTC TTGGTTGAAG CAATGTTGTA GTTTCTATGC CTGCTGAGTA AATAGCCTCA 3058

AGTCCAAGTA CTTGCCCAGA CTAATGATCA AACGTATCCA GGAGTTCCAT ACCAGAGATG 3118

TACTCTTCTC TCCTTTGAAG TACATTGCTG GAAGAGTAAT TGTGTTTGCT AGAGATACTC 3178

CTTCGAACTG CAAAAGAAAT CTCTTGGCTA AGCATATAAT CAAGCCTCAG GTTTTCTTTT 3238

TATTAAATAG CTGCTTGTAA GAAAGTGGAC ACTAAGCATA TACCTCAAAG GGAGACAGAA 3298

TGACTCTGTG CCTTCACTGA TGGAAGTCTG GGTTACAAAT TACATCAGAA GAACCTATCA 3358

TAGTGAAACA TCTCATTCCC CTGGTATAAT CCCTTCTAGA AATACACTTG TGACTCTGAA 3418

ATGTTATAAT CGTGACAACT AGGCTGTTAC AGATACACCA AGTTAAATTT GATAGAGAAA 3478

CCAGGCTTGG AGCCTCATGT CCATAGGGCA AGAGGAAGAT GCTGAGTGTT TAAGGTTGGT 3538

TTGAGCGAAG AACAATACCT TGTGTCACAA AAATGAAAGG AAAAAAGAAA AAAGGAAAGA 3598

AGGAAAGAAA GAGAGAGAAA GAAAAAGAAA GAAAGAAAAA AAAAAAAAA 3647
INFORMATION CONCERNANT LA SEQ ID NO: 5 :
   i) CARACTRERISTIQUE DE LA SEQUENCE :
      A) LONGUEUR 1602 paires de base
      B) TYPE : acide nucléique
      C) NOMBRE DE BRINS : double
      D) CONFIGURATION : linéaire
   ii) TYPE DE MOLECULE : ADN
   vi) ORIGINE : rat
   ix) CARACTERISTIQUE
      A) NOM/CLE : DRASIC
      B) LOCALISATION : 1 .. 1602
   xi) DESCRIPTION DE LA SEQUENCE : SEQ ID NO:5 : 
ATG AAA CCT CGC TCC GGA CTG GAG GAG GCC CAG CGG CGA CAG GCC TCA 48
Met Lys Pro Arg Ser Gly Leu Glu Glu Ala Gln Arg Arg Gln Ala Ser
1               5                   10                  15

GAC ATC CGG GTG TTT GCC AGC AGC TGC ACA ATG CAT GGT CTG GGC CAC 96
Asp Ile Arg Val Phe Ala Ser Ser Cys Thr Met His Gly Leu Gly His
            20                  25                  30

ATC TTT GGC CCT GGA GGC CTG ACC CTG CGC CGA GGG CTG TGG GCC ACA 144
Ile Phe Gly Pro Gly Gly Leu Thr Leu Arg Arg Gly Leu Trp Ala Thr
        35                  40                  45

GCT GTG CTC CTG TCG CTG GCG GCC TTC CTC TAC CAG GTG GCT GAG CGG 192
Ala Val Leu Leu Ser Leu Ala Ala Phe Leu Tyr Gln Val Ala Glu Arg
    50                  55                  60

GTT CGC TAC TAT GGG GAG TTC CAC CAT AAG ACC ACC CTG GAT GAG CGT 240
Val Arg Tyr Tyr Gly Glu Phe His His Lys Thr Thr Leu Asp Glu Arg
65                  70                  75                  80

GAG AGC CAC CAG CTC ACC TTC CCA GCT GTG ACT CTG TGT AAT ATC AAC 288
Glu Ser His Gln Leu Thr Phe Pro Ala Val Thr Leu Cys Asn Ile Asn
                85                  90                  95

CCA CTG CGC CGC TCA CGC CTC ACA CCC AAT GAC TTG CAC TGG GCT GGA 336
Pro Leu Arg Arg Ser Arg Leu Thr Pro Asn Asp Leu His Trp Ala Gly
            100                 105                 110

ACA GCG CTG CTG GGC CTG GAC CCT GCT GAA CAT GCT GCC TAC CTT CGT 384
Thr Ala Leu Leu Gly Leu Asp Pro Ala Glu His Ala Ala Tyr Leu Arg
        115                 120                 125

GCA CTG GGC CAG CCC CCC GCA CCA CCT GGC TTC ATG CCC AGT CCG ACC 432
Ala Leu Gly Gln Pro Pro Ala Pro Pro Gly Phe Met Pro Ser Pro Thr
    130                 135                 140

TTT GAC ATG GCA CAA CTC TAC GCC AGA GCC GGC CAC TCC CTT GAG GAC 480
Phe Asp Met Ala Gln Leu Tyr Ala Arg Ala Gly His Ser Leu Glu Asp
145                 150                 155                 160
 
ATG TTG TTG GAT TGC CGA TAC CGT GGC CAG CCC TGT GGG CCT GAG AAC 528
Met Leu Leu Asp Cys Arg Tyr Arg Gly Gln Pro Cys Gly Pro Glu Asn
                165                 170                 175

TTC ACA GTG ATC TTT ACT CGA ATG GGG CAA TGC TAC ACC TTC AAC TCT 576
Phe Thr Val Ile Phe Thr Arg Met Gly Gln Cys Tyr Thr Phe Asn Ser
            180                 185                 190

GGT GCC CAC GGT GCA GAG CTG CTC ACC ACT CCA AAG GGT GGT GCT GGC 624
Gly Ala His Gly Ala Glu Leu Leu Thr Thr Pro Lys Gly Gly Ala Gly
        195                 200                 205

AAC GGA CTG GAG ATT ATG CTA GAT GTA CAG CAA GAG GAG TAT CTG CCC 672
Asn Gly Leu Glu Ile Met Leu Asp Val Gln Gln Glu Glu Tyr Leu Pro
    210                 215                 220

ATC TGG AAG GAC ATG GAA GAG ACC CCG TTT GAG GTG GGG ATC CGA GTG 720
Ile Trp Lys Asp Met Glu Glu Thr Pro Phe Glu Val Gly Ile Arg Val
225                 230                 235                 240

CAG ATT CAC AGC CAG GAT GAG CCC CCT GCC ATT GAC CAG CTG GGC TTC 768
Gln Ile His Ser Gln Asp Glu Pro Pro Ala Ile Asp Gln Leu Gly Phe
                245                 250                 255

GGG GCA GCC CCA GGC CAT CAG ACT TTT GTG TCC TGT CAG CAG CAG CAA 816
Gly Ala Ala Pro Gly His Gln Thr Phe Val Ser Cys Gln Gln Gln Gln
            260                 265                 270

CTG AGT TTC CTG CCA CCA CCC TGG GGT GAC TGC AAT ACC GCA TCT TTG 864
Leu Ser Phe Leu Pro Pro Pro Trp Gly Asp Cys Asn Thr Ala Ser Leu
        275                 280                 285

GAT CCC GAC GAC TTT GAT CCA GAG CCC TCT GAT CCC TTG GGT TCC CCC 912
Asp Pro Asp Asp Phe Asp Pro Glu Pro Ser Asp Pro Leu Gly Ser Pro
    290                 295                 300

AGA CCC AGA CCC AGC CCT CCT TAT AGT TTA ATA GGT TGT CGC CTG GCC 960
Arg Pro Arg Pro Ser Pro Pro Tyr Ser Leu Ile Gly Cys Arg Leu Ala
305                 310                 315                 320

TGT GAG TCT CGC TAT GTG GCT CGG AAG TGT GGC TGT CGA ATG ATG CAT 1008
Cys Glu Ser Arg Tyr Val Ala Arg Lys Cys Gly Cys Arg Met Met His
                325                 330                 335

ATG CCT GGA AAC TCC CCA GTG TGC AGC CCC CAG CAG TAC AAG GAC TGC 1056
Met Pro Gly Asn Ser Pro Val Cys Ser Pro Gln Gln Tyr Lys Asp Cys
            340                 345                 350

GCC AGC CCA GCT CTG GAC GCT ATG CTG CGA AAG GAC ACG TGT GTC TGC 1104
Ala Ser Pro Ala Leu Asp Ala Met Leu Arg Lys Asp Thr Cys Val Cys
        355                 360                 365

CCC AAC CCG TGC GCT ACT ACA CGC TAT GCC AAG GAG CTC TCC ATG GTG 1152
Pro Asn Pro Cys Ala Thr Thr Arg Tyr Ala Lys Glu Leu Ser Met Val
    370                 375                 380

CGG ATT CCC AGC CGC GCG TCA GCT CGC TAC CTG GCC CGG AAA TAC AAC 1200
Arg Ile Pro Ser Arg Ala Ser Ala Arg Tyr Leu Ala Arg Lys Tyr Asn
385                 390                 395                 400
 
CGC AGC GAG TCC TAC ATT ACG GAG AAT GTA CTG GTT CTG GAT ATC TTC 1248
Arg Ser Glu Ser Tyr Ile Thr Glu Asn Val Leu Val Leu Asp Ile Phe
                405                 410                 415

TTT GAG GCC CTC AAC TAT GAA GCG GTG GAA CAA AAG GCG GCC TAT GAA 1296
Phe Glu Ala Leu Asn Tyr Glu Ala Val Glu Gln Lys Ala Ala Tyr Glu
            420                 425                 430

GTG TCG GAG CTG CTG GGA GAC ATT GGG GGA CAG ATG GGA CTG TTT ATT 1344
Val Ser Glu Leu Leu Gly Asp Ile Gly Gly Gln Met Gly Leu Phe Ile
        435                 440                 445

GGA GCA AGC CTG CTT ACC ATC CTT GAG ATC CTC GAC TAT CTC TGT GAG 1392
Gly Ala Ser Leu Leu Thr Ile Leu Glu Ile Leu Asp Tyr Leu Cys Glu
    450                 455                 460

GTT TTC CAA GAC AGA GTC CTG GGG TAT TTC TGG AAC AGA AGG AGC GCT 1440
Val Phe Gln Asp Arg Val Leu Gly Tyr Phe Trp Asn Arg Arg Ser Ala
465                 470                 475                 480

CAA AAG CGC TCT GGC AAC ACT CTG CTC CAG GAA GAG TTG AAT GGC CAT 1488
Gln Lys Arg Ser Gly Asn Thr Leu Leu Gln Glu Glu Leu Asn Gly His
                485                 490                 495

CGA ACA CAT GTT CCC CAC CTC AGC CTA GGG CCC AGG CCT CCT ACC ACT 1536
Arg Thr His Val Pro His Leu Ser Leu Gly Pro Arg Pro Pro Thr Thr
            500                 505                 510

CCC TGT GCT GTC ACC AAG ACA CTC TCT GCC TCC CAC CGT ACC TGT TAC 1584
Pro Cys Ala Val Thr Lys Thr Leu Ser Ala Ser His Arg Thr Cys Tyr
        515                 520                 525

CTC GTC ACA AGG CTC TAG 1602
Leu Val Thr Arg Leu *
530 533
INFORMATION CONCERNANT LA SEO ID NO:6 :
   i) CARACTRERISTIQUE DE LA SEQUENCE:
      A) LONGUEUR 1948 paires de base
      B) TYPE : acide nucléique
      C) NOMBRE DE BRINS : double
      D) CONFIGURATION : linéaire
   ii) TYPE DE MOLECULE : ADN
   vi) ORIGINE : rat
   ix) CARACTERISTIQUE
      A) NOM/CLE : MDEG2
      B) LOCALISATION : 16 .. 1707
   xi) DESCRIPTION DE LA SEQUENCE : SEQ ID NO:6 :

CCTCGGGCTG AATGA ATG AGC CGG AGC GGC GGA GCC CGG CTG CCC GCG ACC 51
Met Ser Arg Ser Gly Gly Ala Arg Leu Pro Ala Thr
1               5                   10

GCG CTC AGC GGC CCG GGA CGC TTC CGT ATG GCC CGC GAG CAG CCG GCG 99
Ala Leu Ser Gly Pro Gly Arg Phe Arg Met Ala Arg Glu Gln Pro Ala
        15                  20                  25

CCC GTG GCG GTG GCG GCA GCT AGG CAG CCC GGA GGA GAC CGG AGC GGC 147
Pro Val Ala Val Ala Ala Ala Arg Gln Pro Gly Gly Asp Arg Ser Gly
    30                  35                  40

GAT CCG GCG CTG CAG GGG CCA GGG GTC GCC CGC AGG GGG CGG CCG TCC 195
Asp Pro Ala Leu Gln Gly Pro Gly Val Ala Arg Arg Gly Arg Pro Ser
45                  50                  55                  60

CTG AGT CGC ACT AAA TTG CAC GGG CTG CGG CAC ATG TGC GCG GGG CGC 243
Leu Ser Arg Thr Lys Leu His Gly Leu Arg His Met Cys Ala Gly Arg
                65                  70                  75

ACG GCG GCG GGA GGC TCT TTC CAG CGA CGG GCG CTG TGG GTG CTG GCC 291
Thr Ala Ala Gly Gly Ser Phe Gln Arg Arg Ala Leu Trp Val Leu Ala
            80                  85                  90

TTC TGC ACG TCC CTC GGC TTG CTG CTG TCC TGG TCC TCG AAC CGC CTG 339
Phe Cys Thr Ser Leu Gly Leu Leu Leu Ser Trp Ser Ser Asn Arg Leu
        95                  100                 105

CTC TAC TGG CTC AGC TTC CCG TCA CAC ACA CGA GTG CAC CGT GAG TGG 387
Leu Tyr Trp Leu Ser Phe Pro Ser His Thr Arg Val His Arg Glu Trp
    110                 115                 120

AGC CGC CAG CTG CCG TTC CCC GCC GTC ACC GTG TGC AAC AAC AAC CCC 435
Ser Arg Gln Leu Pro Phe Pro Ala Val Thr Val Cys Asn Asn Asn Pro
125                 130                 135                 140

CTG CGC TTC CCG CGC CTC TCC AAG GGG GAC CTC TAC TAC GCG GGC CAC 483
Leu Arg Phe Pro Arg Leu Ser Lys Gly Asp Leu Tyr Tyr Ala Gly His
                145                 150                 155

TGG CTA GGG CTG CTG CTT CCC AAC CGC ACC GCG CGC CCG CTG GTC AGC 531
Trp Leu Gly Leu Leu Leu Pro Asn Arg Thr Ala Arg Pro Leu Val Ser
            160                 165                 170

GAG CTG CTG CGG GGC GAC GAG CCG CGC CGC CAG TGG TTC CGC AAA CTG 579
Glu Leu Leu Arg Gly Asp Glu Pro Arg Arg Gln Trp Phe Arg Lys Leu
        175                 180                 185

GCC GAC TTC CGC CTC TTC CTG CCG CCG CGC CAC TTC GAG GGC ATC AGC 627
Ala Asp Phe Arg Leu Phe Leu Pro Pro Arg His Phe Glu Gly Ile Ser
    190                 195                 200

GCT GCC TTC ATG GAC CGT TTG GGC CAC CAG CTG GAG GAT ATG CTG CTC 675
Ala Ala Phe Met Asp Arg Leu Gly His Gln Leu Glu Asp Met Leu Leu
205                 210                 215                 220

TCC TGC AAG TAC CGG GGC GAG CTC TGT GGC CCG CAC AAC TTC TCC TCA 723
Ser Cys Lys Tyr Arg Gly Glu Leu Cys Gly Pro His Asn Phe Ser Ser
                225                 230                 235

GTG TTT ACA AAA TAC GGG AAG TGT TAC ATG TTT AAC TCA GGC GAG GAT 771
Val Phe Thr Lys Tyr Gly Lys Cys Tyr Met Phe Asn Ser Gly Glu Asp
            240                 245                 250

GGC AAG CCG CTG CTC ACC ACG GTC AAG GGG GGG ACG GGC AAC GGG CTG 819
Gly Lys Pro Leu Leu Thr Thr Val Lys Gly Gly Thr Gly Asn Gly Leu
        255                 260                 265

GAG ATC ATG CTG GAC ATT CAG CAA GAT GAG TAC CTG CCC ATC TGG GGA 867
Glu Ile Met Leu Asp Ile Gln Gln Asp Glu Tyr Leu Pro Ile Trp Gly
    270                 275                 280

GAG ACA GAG GAA ACA ACG TTT GAA GCA GGA GTG AAG GTT CAG ATC CAC 915
Glu Thr Glu Glu Thr Thr Phe Glu Ala Gly Val Lys Val Gln Ile His
285                 290                 295                 300

AGT CAG TCT GAG CCG CCT TTC ATC CAA GAG CTG GGC TTT GGG GTG GCT 963
Ser Gln Ser Glu Pro Pro Phe Ile Gln Glu Leu Gly Phe Gly Val Ala
                305                 310                 315

CCG GGG TTC CAG ACC TTC GTG GCC ACA CAA GAG CAG AGG CTC ACA TAT 1011
Pro Gly Phe Gln Thr Phe Val Ala Thr Gln Glu Gln Arg Leu Thr Tyr
            320                 325                 330

CTG CCC CCA CCA TGG GGG GAG TGC CGG TCC TCA GAG ATG GGA CTC GAC 1059
Leu Pro Pro Pro Trp Gly Glu Cys Arg Ser Ser Glu Met Gly Leu Asp
        335                 340                 345

TTC TTT CCT GTT TAC AGC ATC ACA GCC TGT CGG ATT GAC TGT GAG ACC 1107
Phe Phe Pro Val Tyr Ser Ile Thr Ala Cys Arg Ile Asp Cys Glu Thr
    350                 355                 360

CGC TAC ATC GTG GAG AAC TGT AAC TGC CGC ATG GTC CAC ATG CCA GGG 1155
Arg Tyr Ile Val Glu Asn Cys Asn Cys Arg Met Val His Met Pro Gly
365                 370                 375                 380

GAC GCC CCT TTC TGC ACC CCT GAG CAG CAC AAG GAG TGT GCA GAG CCT 1203
Asp Ala Pro Phe Cys Thr Pro Glu Gln His Lys Glu Cys Ala Glu Pro
                385                 390                 395

GCC CTC GGT CTA CTG GCA GAA AAG GAC AGC AAT TAC TGT CTC TGC AGG 1251
Ala Leu Gly Leu Leu Ala Glu Lys Asp Ser Asn Tyr Cys Leu Cys Arg
            400                 405                 410

ACA CCC TGC AAC CTG ACA CGC TAC AAC AAA GAG CTC TCC ATG GTG AAG 1299
Thr Pro Cys Asn Leu Thr Arg Tyr Asn Lys Glu Leu Ser Met Val Lys
        415                 420                 425

ATC CCC AGC AAG ACG TCA GCC AAG TAC TTA GAG AAG AAA TTT AAC AAA 1347
Ile Pro Ser Lys Thr Ser Ala Lys Tyr Leu Glu Lys Lys Phe Asn Lys
    430                 435                 440

TCG GAA AAA TAT ATC TCA GAG AAC ATT CTT GTT CTG GAC ATA TTT TTT 1395
Ser Glu Lys Tyr Ile Ser Glu Asn Ile Leu Val Leu Asp Ile Phe Phe
445                 450                 455                 460

GAG GCG CTC AAT TAC GAA ACA ATT GAA CAG AAG AAG GCG TAT GAA GTT 1443
Glu Ala Leu Asn Tyr Glu Thr Ile Glu Gln Lys Lys Ala Tyr Glu Val
                465                 470                 475

GCT GCC TTA CTT GGT GAC ATC GGT GGT CAG ATG GGA CTG TTC ATT GGT 1491
Ala Ala Leu Leu Gly Asp Ile Gly Gly Gln Met Gly Leu Phe Ile Gly
            480                 485                 490

GCT AGT CTC CTC ACA ATA CTA GAG CTC TTT GAT TAT ATT TAT GAG CTG 1539
Ala Ser Leu Leu Thr Ile Leu Glu Leu Phe Asp Tyr Ile Tyr Glu Leu
        495                 500                 505

ATC AAA GAG AAG CTA TTA GAC CTG CTT GGC AAA GAA GAA GAG GAA GGG 1587
Ile Lys Glu Lys Leu Leu Asp Leu Leu Gly Lys Glu Glu Glu Glu Gly
    510                 515                 520

AGC CAC GAT GAG AAC ATG AGC ACC TGT GAC ACA ATG CCA AAC CAC TCT 1635
Ser His Asp Glu Asn Met Ser Thr Cys Asp Thr Met Pro Asn His Ser
525                 530                 535                 540

GAA ACC ATC AGC CAC ACT GTG AAC GTG CCC CTG CAG ACA GCT TTG GGC 1683
Glu Thr Ile Ser His Thr Val Asn Val Pro Leu Gln Thr Ala Leu Gly
                545                 550                 555

ACC CTG GAG GAG ATT GCC TGC TGA CACCTCTCAG GCAACGCAGC ACCTCCAAAC 1737
Thr Leu Glu Glu Ile Ala Cys *
560 563

AGACCTTAAA GGCCCAAGAC CTAGGACAGG AGACAGCAAG CGCAGGTGGG ATCGCCCCTG 1797
ACGACTGAAA GAAGCAGAGC CCCCCATATG CACACATTGC GAACTTCTGC CAAACCTCAC 1857
CTGGCCACAT CTGACATGAA CCGTCCCGGG CCCTGCGTCA TGTCCCTCGC AGGACCGATG 1917
AGTCGCACTC CGGAACTGTC CAAGAACTAA C 1948

## Revendications

1. Protéine constituant un canal cationique neuronal de mammifère sensible à l'amiloride et activé par les protons, **caractérisée en ce qu'**elle consiste en l'une des séquences en acides aminés représentées dans la liste de séquences en annexe sous le numéro SEQ ID No : 1 ou SEQ ID No : 2.

2. Canal cationique hybride, **caractérisé en ce qu'**il est constitué de l'association d'une première protéine constituant un canal ionique activé par les protons selon la revendication 1 ou consistant en l'une des séquences en acides aminés représentées dans la liste de séquences en annexe sous le numéro SEQ ID No : 3, SEQ ID No : 4 ou SEQ ID No : 5 ou SEQ ID No : 6, avec une seconde protéine constituant un canal ionique activé ou non par les protons.

3. Canal cationique hybride selon la revendication 2, **caractérisé en ce que** la seconde protéine constitue un canal ionique activé par les protons, consistant en l'une des séquences en acides aminés représentées dans la liste de séquences en annexe sous le numéro SEQ ID No : 3 ou SEQ ID No : 6.

4. Canal cationique hybride selon l'une quelconque des revendications 2 ou 3, **caractérisé en ce que** ladite première protéine est une protéine dont la séquence en acides aminés est représentée dans la liste de séquence en annexe sous le numéro SEQ ID No : 1, SEQ ID No : 2, SEQ ID No : 3, SEQ ID No : 4 ou SEQ ID No : 5 et la seconde protéine est une protéine dont la séquence en acides aminés est représentée dans la liste de séquence en annexe sous le numéro SEQ ID No : 3 ou SEQ ID No : 6.

5. Anticorps monoclonal ou polyclonal dirigé contre au moins une protéine selon la revendication 1 et/ou contre au moins un canal hybride selon l'une quelconque des revendications 2 à 4.

6. Molécule d'acide nucléique comprenant ou constituée par une séquence nucléique codant pour une protéine constituant un canal cationique selon la revendication 1 ou un canal hybride selon l'une quelconque des revendications 2 à 4.

7. Molécule d'acide nucléique selon la revendication 6 comprenant ou constituée par la séquence nucléique comprise entre les nucléotides 123 et 1700 de la séquence représentée dans la liste de séquences en annexe sous le numéro SEQ ID No : 1, ou sa séquence complémentaire.

8. Molécule d'acide nucléique selon la revendication 6 comprenant ou constituée par la séquence nucléique comprise entre les nucléotides 1 et 1542 de la séquence représentée dans la liste de séquences en annexe sous le numéro SEQ ID No : 2, ou sa séquence complémentaire.

9. Molécule d'acide nucléique selon la revendication 6 comprenant la séquence nucléique comprise entre les nucléotides 109 et 1785 de la séquence représentée dans la liste de séquences en annexe sous le numéro SEQ ID No : 4, ou sa séquence complémentaire.

10. Molécule d'acide nucléique selon la revendication 6 comprenant la séquence nucléique comprise entre les nucléotides 1 et 1602 de la séquence représentée dans la liste de séquences en annexe sous le numéro SEQ ID No : 5, ou sa séquence complémentaire.

11. Vecteur comprenant au moins une molécule d'acide nucléique selon l'une quelconque des revendications 6 à 10, avantageusement associée à des séquences de contrôle.

12. Procédé de production d'une protéine constituant un canal ionique selon la revendication 1 ou un canal hybride selon l'une quelconque des revendications 2 à 4, **caractérisé en ce qu'**il consiste :
- à transférer une molécule d'acide nucléique selon l'une des revendications 6 à 10 ou un vecteur selon la revendication 11 dans un hôte cellulaire,
- à cultiver ledit hôte cellulaire dans des conditions permettant la production de la protéine constituant le canal ionique,
- à isoler, par tous moyens appropriés les protéines constituant les canaux ioniques.

13. Procédé d'expression d'une protéine constituant un canal ionique selon la revendication 1 ou un canal hybride selon l'une quelconque des revendications 2 à 4, dans un hôte cellulaire, **caractérisé en ce qu'**il consiste à :
- transférer une molécule d'acide nucléique selon l'une des revendications 6 à 10 ou un vecteur selon la revendication 11 dans ledit hôte cellulaire,
- cultiver ledit hôte cellulaire dans des conditions permettant la production de la protéine constituant le canal ionique.

14. Procédé selon l'une des revendications 12 ou 13, **caractérisé en ce que** l'hôte cellulaire est choisi parmi les procaryotes ou les eucaryotes et notamment parmi les bactéries, les levures, les cellules de mammifères, de plantes ou d'insectes.

15. Cellule transformée par un acide nucléique selon l'une quelconque des revendications 6 à 11 exprimant des canaux cationiques neuronaux de mammifère sensibles à l'amiloride et activés par les protons selon l'une des revendications 1 à 4.

16. Procédé de criblage de substances capables de moduler l'activité de canaux ioniques neuronaux de mammifère, **caractérisé en ce que** :
- l'on met en contact des quantités variables d'une substance à tester avec des cellules selon la revendication 15 exprimant des canaux cationiques neuronaux de mammifère sensibles à l'amiloride et activés par les protons, puis
- l'on mesure, par tous moyens appropriés, les effets éventuels de ladite substance sur les courants des canaux cationiques sensibles à l'amiloride et activés par les protons,
lesdites cellules exprimant des canaux cationiques neuronaux de mammifère sensibles à l'amiloride et activés par les protons choisis dans le groupe comprenant les canaux selon l'une des revendications 1 à 4 ou les canaux consistant en une séquence en acides aminés représentée dans la liste de séquences en annexe sous les numéros SEQ ID No : 3 ou SEQ ID No : 6.

17. Procédé de criblage de substances capables de moduler l'activité de canaux ioniques neuronaux de mammifère, **caractérisé en ce que** l'on met en contact des quantités variables d'une substance à tester avec des cellules selon la revendication 15, puis l'on mesure, par tous moyens appropriés, les effets éventuels de ladite substance sur les courants des canaux cationiques sensibles à l'amiloride et activés par les protons.

18. Procédé selon la revendication 16 ou 17 appliqué au criblage de substances capables de moduler la perception de l'acidité, tant en ce qui concerne la nociception que la transduction du goût.

## Claims

1. Protein forming an amiloride-sensitive and proton-activated mammalian neuronal cation channel, **characterised in that** it consists of any of the amino acid sequences represented in the appended list of sequences under the number SEQ ID No: 1 or SEQ ID No: 2.

2. Hybrid cation channel, **characterised in that** it consists of the association of a first protein forming a proton-activated ion channel according to claim 1 or consisting of any of the amino acid sequences represented in the appended list of sequences under the number SEQ ID No: 3, SEQ ID No: 4 or SEQ ID No: 5 or SEQ ID No: 6, with a second protein forming a ion channel activated or not by protons.

3. Hybrid cation channel according to claim 2, **characterised in that** the second protein forms a proton-activated ion channel, consisting of any of the amino acid sequences represented in the list of sequences appended under the number SEQ ID No: 3 or SEQ ID No: 6.

4. Hybrid cation channel according to any of claims 2 or 3, **characterised in that** said first protein is a protein which amino acid sequence is represented in the appended list of sequences under the number SEQ ID No: 1, SEQ ID No: 2 or SEQ ID No: 3, SEQ ID No: 4 or SEQ ID No: 5 and the second protein is a protein which amino acid sequence is represented in the appended list of sequences under the number SEQ ID No: 3 or SEQ ID No: 6.

5. Monoclonal or polyclonal antibody targeted against at least one protein according to claim 1 and/or against at least one hybrid channel according to any of claims 2 to 4.

6. Nucleic acid molecule comprising or consisting of a nucleic sequence encoding a protein forming a cation channel according to claim 1 or a hybrid channel according to any of claims 2 to 4.

7. Nucleic acid molecule according to claim 6 comprising or consisting of the nucleic acid sequence between nucleotides 123 and 1700 of the sequence represented in the appended list of sequences under the number SEQ ID No: 1, or the complementary sequence thereof.

8. Nucleic acid molecule according to claim 6 comprising or consisting of the nucleic sequence between nucleotides 1 and 1542 of the sequence represented in the appended list of sequences under the number SEQ ID No: 2, or the complementary sequence thereof.

9. Nucleic acid molecule according to claim 6 comprising or consisting of the nucleic sequence between nucleotides 109 and 1785 of the sequence represented in the appended list of sequences under the number SEQ ID No: 4, or the complementary sequence thereof.

10. Nucleic acid molecule according to claim 6 comprising or consisting of the nucleic sequence between nucleotides 1 and 1602 of the sequence represented in the appended list of sequences under the number SEQ ID No: 5, or the complementary sequence thereof.

11. Vector comprising at least one nucleic acid molecule according to any of claims 6 to 10, advantageously associated with control sequences.

12. Method for production of a protein forming an ion channel according to claim 1 or a hybrid channel according to any of claims 2 to 4, **characterised in that** it consists of:
- transferring a nucleic acid molecule according to any of claims 6 to 10 or a vector according to claim 11 in a cell host,
- culturing said cell host under conditions enabling the production of the protein forming the ion channel,
- isolating, by any suitable means, the proteins forming the ion channels.

13. Method for expression of a protein forming an ion channel according to claim 1 or a hybrid channel according to any of claims 2 to 4, in a cell host, **characterised in that** it consists of:
- transferring a nucleic acid molecule according to any of claims 6 to 10 or a vector according to claim 11 into said cell host,
- culturing said cell host under conditions enabling the production of the protein forming the ion channel.

14. Method according to any of claims 12 or 13, **characterised in that** the cell host is selected from prokaryotes or eukaryotes and particularly from bacteria, yeasts, mammalian, plant or insect cells.

15. Cell transformed by a nucleic acid according to any of claims 6 to 11 expressing amiloride-sensitive and proton-activated neuronal cation channels according to any of claims 1 to 4.

16. Method for screening of substances capable of modulating the activity of mammalian neuronal ion channels, **characterised in that**:
- variable quantities of a test substance are placed in contact with cells according to claim 15 expressing amiloride-sensitive and proton-activated mammalian neuronal cation channels, and then
- any suitable means are used to measure the potential effects of said substance on the flows of the amiloride-sensitive and proton-activated mammalian neuronal cation channels,
said cells expressing amiloride-sensitive and proton-activated mammalian neuronal cation channels selected from the group comprising the channels according to any of claims 1 to 4 or the channels consisting of an amino acid sequence represented in the appended list of sequences under the numbers SEQ ID No: 3 or SEQ ID No: 6.

17. Method for screening of substances capable of modulating the activity of mammalian neuronal ion channels, **characterised in that** variable quantities of a test substance are placed in contact with cells according to claim 15, and any suitable means are used to measure the potential effects of said substance on the flows of the amiloride-sensitive and proton-activated mammalian neuronal cation channels are measured.

18. Method according to claim 16 or 17 applied to the screening of substances capable of modulating the perception of acidity, both in terms of taste nociception and transduction.

## Patentansprüche

1. Protein, einen auf Amilorid sensiblen und von den Protonen aktivierten neuronalen Säugetier-Kationenkanal bildend, **dadurch gekennzeichnet**, das es aus einer seiner Aminosäuresequenzen, die in der Sequenzliste in der Anlage unter der Nummer SEQ ID Nr. 1 oder SEQ ID Nr. 2 dargestellt sind, besteht.

2. Hybrid-Kationenkanal, **dadurch gekennzeichnet, dass** er aus der Verbindung eines ersten Proteins, das einen von den Protonen nach Anspruch 1 aktivierten Ionenkanal bildet oder aus einer der Aminosäuresequenzen, die in der Sequenzliste in der Anlage unter der Nummer SEQ ID Nr. 3, SEQ ID Nr. 4 oder SEQ ID Nr. 5 oder SEQ ID Nr. 6 dargestellt sind, besteht, mit einem zweiten Protein, das einen Ionenkanal bildet, der von den Protonen aktiviert ist oder nicht, gebildet wird.

3. Hybrid-Kationenkanal nach Anspruch 2, **dadurch gekennzeichnet, dass** das zweite Protein einen von den Protonen aktivierten Ionenkanal bildet, der aus einer der Aminosäuresequenzen besteht, die in der Sequenzliste in der Anlage unter der Nummer SEQ ID Nr. 3 oder SEQ ID Nr. 6 dargestellt sind.

4. Hybrid-Kationenkanal nach einem der Ansprüche 2 oder 3, **dadurch gekennzeichnet, dass** das erste Protein ein Protein ist, dessen Aminosäuresequenz in der Sequenzliste in der Anlage unter der Nummer SEQ ID Nr. 1, SEQ ID Nr. 2, SEQ ID Nr. 3, SEQ ID Nr. 4 oder SEQ ID Nr. 5 dargestellt ist, und das zweite Protein ein Protein ist, dessen Aminosäuresequenz in der Sequenzliste in der Anlage unter der Nummer SEQ ID Nr. 3 oder SEQ ID Nr. 6 dargestellt ist.

5. Monoklonaler oder polyklonaler Antikörper, gegen mindestens ein Protein nach Anspruch 1 und/oder gegen mindestens einen Hybridkanal nach einem der Ansprüche 2 bis 4 gerichtet.

6. Nukleinsäuremolekül, eine Nukleinsequenz umfassend oder davon gebildet, die für ein Protein kodiert, das einen Kationenkanal nach Anspruch 1 oder einen Hybridkanal nach einem der Ansprüche 2 bis 4 bildet.

7. Nukleinsäuremolekül nach Anspruch 6, die Nukleinsequenz umfassend oder davon gebildet, die zwischen den Nukleotiden 123 und 1700 der Sequenz eingeschlossen ist, die in der Sequenzliste in der Anlage unter der Nummer SEQ ID Nr. 1 dargestellt ist, oder ihre komplementäre Sequenz.

8. Nukleinsäuremolekül nach Anspruch 6, die Nukleinsequenz umfassend oder davon gebildet, die zwischen den Nukleotiden 1 und 1542 der Sequenz eingeschlossen ist, die in der Sequenzliste in der Anlage unter der Nummer SEQ ID Nr. 2 dargestellt ist, oder ihre komplementäre Sequenz.

9. Nukleinsäuremolekül nach Anspruch 6, die Nukleinsequenz umfassend, die zwischen den Nukleotiden 109 und 1785 der Sequenz eingeschlossen ist, die in der Sequenzliste in der Anlage unter der Nummer SEQ ID Nr. 4 dargestellt ist, oder ihre komplementäre Sequenz.

10. Nukleinsäuremolekül nach Anspruch 6, die Nukleinsequenz umfassend, die zwischen den Nukleotiden 1 und 1602 der Sequenz eingeschlossen ist, die in der Sequenzliste in der Anlage unter der Nummer SEQ ID Nr. 5 dargestellt ist, oder ihre komplementäre Sequenz.

11. Vektor, der mindestens ein Nukleinsäuremolekül nach einem der Ansprüche 6 bis 10 umfasst, in vorteilhafter Weise mit Kontrollsequenzen verbunden.

12. Verfahren zur Herstellung eines Proteins, das einen Ionenkanal nach Anspruch 1 oder einen Hybridkanal nach einem der Ansprüche 2 bis 4 bildet, **dadurch gekennzeichnet**, das es darin besteht:
- ein Nukleinsäuremolekül nach einem der Ansprüche 6 bis 10 oder einen Vektor nach Anspruch 11 in eine Wirtszelle zu übertragen,
- diese Wirtszelle unter Bedingungen zu kultivieren, die die Herstellung des den Ionenkanal bildenden Proteins gestatten,
- die die Ionenkanäle bildenden Proteine mit allen geeigneten Mitteln zu isolieren.

13. Verfahren zur Expression eines Proteins, das einen Ionenkanal nach Anspruch 1 oder einen Hybridkanal nach einem der Ansprüche 2 bis 4 bildet, in einer Wirtszelle, **dadurch gekennzeichnet, dass** es darin besteht:
- ein Nukleinsäuremolekül nach einem der Ansprüche 6 bis 10 oder einen Vektor nach Anspruch 11 in die Wirtszelle zu übertragen,
- diese Wirtszelle unter Bedingungen zu kultivieren, die die Herstellung des den Ionenkanal bildenden Proteins gestatten.

14. Verfahren nach einem der Ansprüche 12 oder 13, **dadurch gekennzeichnet, dass** die Wirtszelle aus den Prokaryoten oder Eukaryoten und vor allem aus den Bakterien, den Hefen, den Säugetier-, Pflanzen- oder Insektenzellen ausgewählt ist.

15. Durch eine Nukleinsäure nach einem der Ansprüche 6 bis 11 transformierte Zelle, die auf Amilorid sensible und von den Protonen nach einem der Ansprüche 1 bis 4 aktivierte neuronale Säugetier-Kationenkanäle exprimiert.

16. Screeningverfahren für Substanzen, die in der Lage sind, die Aktivität von neuronalen Säugetier-Ionenkanälen zu modulieren, **dadurch gekennzeichnet, dass**:
- variable Mengen einer Testsubstanz mit Zellen nach Anspruch 15, die auf Amilorid sensible und von den Protonen aktivierte neuronale Säugetier-Kationenkanäle exprimieren, in Kontakt gebracht werden,
- die eventuellen Wirkungen dieser Substanz auf die Ströme der auf Amilorid sensiblen und von den Protonen aktivierten Kationenkanäle mit allen geeigneten Mitteln gemessen werden,
wobei diese Zellen auf Amilorid sensible und von den Protonen aktivierte neuronale Säugetier-Kationenkanäle exprimieren, die aus der Gruppe ausgewählt sind, die die Kanäle nach einem der Ansprüche 1 bis 4 umfassen oder die Kanäle, die aus einer Aminosäuresequenz bestehen, die in der Sequenzliste in der Anlage unter den Nummern SEQ ID Nr. 3 oder SEQ ID Nr. 6 dargestellt ist.

17. Screeningverfahren für Substanzen, die in der Lage sind, die Aktivität von neuronalen Säugetier-Ionenkanälen zu modulieren, **dadurch gekennzeichnet, dass** variable Mengen einer Testsubstanz mit Zellen nach Anspruch 15 in Kontakt gebracht werden und danach die eventuellen Wirkungen dieser Substanz auf die Ströme der auf Amilorid sensiblen und von den Protonen aktivierten Kationenkanäle mit allen geeigneten Mitteln gemessen werden.

18. Verfahren nach Anspruch 16 oder 17, angewendet auf das Screening von Substanzen, die in der Lage sind, die Wahrnehmung des Säuregrads, sowohl die Schmerzempfindung als auch die Transduktion des Geschmacks betreffend, zu modulieren.
